# EUROPEAN PATENT SPECIFICATION

(11) **EP 1 889 068 B1**
(45) Date of publication and mention of the grant of the patent: **11.08.2010**
(21) Application number: 06779952.8
(22) Date of filing: 26.05.2006
(51) Int. Cl.: G01N 33/574

(54) **HEMATOPOIETIC LINEAGE CELL SPECIFIC PROTEIN (HSI) AS A MARKER FOR LYMPHOID MALIGNANCY**
FÜR HÄMATOPOIETISCHE STAMMZELLEN SPEZIFISCHES PROTEIN (HSI) ALS MARKER FÜR MALIGNE LYMPHOME
PROTEINE (HSI) DE LIGNEES DES CELLULES HEMATOPOIETIQUES DESTINE COMME MARQUEUR DE MALIGNITES LYMPHOIDES

(30) Priority: 27.05.2005 GB 0510922
(43) Date of publication of application: 20.02.2008
(73) Proprietor: FONDAZIONE CENTRO SAN RAFFAELE DEL MONTE TABOR, 20132 Milano (IT)
(72) Inventor: CALIGARIS-CAPPIO, Federico Fondazione Centro San Raffaele del Monte Tabor, I-20132 Milan (IT); SCIELZO, Cristina Fondazione Centro San Raffaele del Monte Tabor, I-20132 Milan (IT); GHIA, Paolo, Prospero Fondazione Centro San Raffaele del Monte Tabor, I-20132 Milan (IT); ALESSIO, Massimo Fondazione Centro San Raffaele del Monte Tabor, I-20132 Milan (IT)
(74) Representative: Mallalieu, Catherine Louise
(86) International application number: PCT/IB2006/002175
(87) International publication number: WO 2006/129203

(56) References cited:
- SCIELZO CRISTINA ET AL: "HS1 protein is differentially expressed in chronic lymphocytic leukemia patient subsets with good or poor prognoses" JOURNAL OF CLINICAL INVESTIGATION, vol. 115, no. 6, June 2005 (2005-06), pages 1644-1650, XP002412717 ISSN: 0021-9738
- YAMANASHI YUJI ET AL: "Role of tyrosine phosphorylation of HS1 in B cell antigen receptor-mediated apoptosis" JOURNAL OF EXPERIMENTAL MEDICINE, vol. 185, no. 7, 1997, pages 1387-1392, XP002412719 ISSN: 0022-1007
- FISCHER ULRIKE ET AL: "Expression of the gene for hematopoietic cell specific protein is not restricted to cells of hematopoietic origin" INTERNATIONAL JOURNAL OF MOLECULAR MEDICINE, vol. 15, no. 4, April 2005 (2005-04), pages 611-615, XP002412718 ISSN: 1107-3756
- CHEN YI-RONG ET AL: "Caspase-mediated cleavage of actin-binding and SH3-domain-containing proteins cortactin, HS1, and HIP-55 during apoptosis" BIOCHEMICAL AND BIOPHYSICAL RESEARCH COMMUNICATIONS, vol. 288, no. 4, 9 November 2001 (2001-11-09), pages 981-989, XP002412713 ISSN: 0006-291X
- CONTRI ANTONELLA ET AL: "Chronic lymphocytic leukemia B cells contain anomalous Lyn tyrosine kinase, a putative contribution to defective apoptosis" JOURNAL OF CLINICAL INVESTIGATION, vol. 115, no. 2, February 2005 (2005-02), pages 369-378, XP002412715 ISSN: 0021-9738 cited in the application
- BROADBRIDGE R J ET AL: "The Src homology-2 domains (SH2 domains) of the protein tyrosine kinase p56lck: structure, mechanism and drug design" CURRENT DRUG TARGETS, BENTHAM SCIENCE PUBLISHER,, US, vol. 1, no. 4, December 2000 (2000-12), pages 365-386, XP002382016 ISSN: 1389-4501

## Description

### FIELD OF THE INVENTION

The present invention relates *inter alia* to diagnostic or prognostic markers for and methods of treating diseases such as Chronic Lymphocytic Leukemia, a disease in which B cell receptor stimulation is dysregulated or diseases characterised by B lymphocyte proliferation /accumulation.

### BACKGROUND TO THE INVENTION

Chronic lymphocytic leucemia is an indolent lymphoid malignancy chracterized by the relentless accumulation in the peripheral blood of monoclonal, resting B lymphocytes expressing CD5 on the cell surface. It arises from the bone marrow and infiltrates secondary lymphoid tissues as lymph nodes and spleen. CLL is characterized by lymphocytosis, anemia and thrombocytopenia and typically by immunodeficiency with progressive hypogammaglobulinemia as well as autoimmune manifestations caused by polyclonal autoantibodies.

About 8,100 new cases are found each year in the U.S. alone, mostly on older people over 50 years of age. It currently has no cure.

Yamanashi Y et al, Proc Natl Acad Sci USA (1993) 90(8):3631-5 describes B cell receptor mediated HS 1 phosphorylation catalysed by Syk and Lyn. However, there is no recognition of the role of HS1 as a prognostic or diagnostic tool or its role in potential therapies.

Brunati AM, J Biol Chem (2005) published on 28 March 2005 as Manuscript M412634200 describes that thrombin-induced Tyr-phosphorylation of HS1 in human platelets is sequentially catalysed by Syk and Lyn tyrosine kinases and associated with the cellular migration of the protein. However, no therapeutic or prognostic effect is proposed.

Contri A et al, J. Clin. Invest. (2005) 115(2):369-78 suggests a correlation between high basal Lyn activity and defects in the introduction of apoptosis in leukemic cells. They found addition of the Lyn inhibitors PP2 and SU6656 to leukemic cell cultures restored cell apoptosis, and the treatment of malignant cells with drugs that induce cell apoptosis decreases both activity and amount of the tyrosine kinase. They also suggested a role for Lyn in CLL B cell pathogenesis and identify this tyrosine kinase as a potential therapeutic target, but there is no mention or realisation of that fact that in order to achieve a therapeutic effect a blockage of HS1 might be sufficient and favourable.

Yamanashi Yuji et al.("Role of tyrosine phosphorylation of HS1 in B cell antigen receptor-mediated apoptosis" Journal of Experimental Medicine, vol. 185, no. 7, 1997, pages 1387-1392) disclosed the identification of HS1 as a protein which is phosphorylated during B cell antigen receptor mediated signalling.

The present inventors have found a new method of determining a patient's prognosis in CLL and similar diseases. They have also found a new method of treating such diseases.

### STATEMENTS OF THE INVENTION

According to one aspect of the invention there is provided an *in vitro* use of a phosphorylated form of hematopoietic-lineage-cell-specific-protein-1 (HS1) as a prognostic or diagnostic marker for Chronic Lymphocytic Leukemia (CLL). According to another aspect of the invention there is provided an *in vitro* method of determining the prognosis of an individual suffering from Chronic Lymphocytic Leukemia (CLL) comprising determining whether the individual's HS1 is in a phosphorylated form, and wherein an increased amount of the phosphorylated form relative to the unphosphorylated form indicates a poorer prognosis for the individual relative to a individual which has a decreased amount or same amount of the phosphorylated form relative to the unphosphorylated form.

According to another aspect of the invention there is provided an *in vitro* method of diagnosing Chronic Lymphocytic Leukemia (CLL) in an individual comprising determining whether the individual's HS1 is in a phosphorylated form, and wherein an increased amount of the phosphorylated form relative to a control indicates the presence of Chronic Lymphocytic Leukemia (CLL).

According to another aspect of the invention there is provided a use of an antibody which specifically recognises phosphorylated HS1 for the preparation of a medicament for the treatment of Chronic Lymphocytic Leukemia (CLL).

According to another aspect of the invention there is provided a method of identifying an antagonist of HS 1 phosphorylation for the prognosis or treatment of Chronic Lymphocytic Leukemia (CLL), the method comprising: (a) providing a candidate molecule, (b) incubating the candidate molecule with HS1 in a phosphorylating environment, and (c) detecting whether phosphorylation of HS1 occurs.

According to another aspect of the invention there is provided a method of identifying an agonist of HS1 dephosphorylation for the prognosis or treatment of Chronic Lymphocytic Leukemia (CLL), the method comprising: (a) providing a candidate molecule, (b) incubating the candidate molecule with phosphorylated HS1, and (c) detecting whether dephosphorylation of HS1 occurs.

According to another aspect of the invention there is provided an antibody which specifically recognises phosphorylated HS 1 and which is generatable against an HS 1 sequence which comprises phosphorylated tyrosine 222, and/or 378, and/or 397.

According to another aspect of the invention there is provided an antibody which specifically recognises phosphorylated HS1 for the treatment of Chronic Lymphocytic Leukemia (CLL).

Described herein is the use of a phosphorylated form of hematopoietic-lineage-cell-specific-protein-1(HS1) as a diagnostic or prognostic marker for Lymphoid malignacies, a disease in which B cell receptor stimulation is dysregulated or disease characterised by B lymphocyte proliferation/accumulation.

The invention may be useful in relation to Lymphoid malignancies of B cell origin such as Chronic Lymphocytic Leukemia (CLL), Follicular Lymphoma or Multiple Myeloma.

Diseases which are characterised by B lymphocyte proliferation/accumulation include autoimmune diseases and graft-versus-host disease.

Described herein is a method of determining the prognosis of an individual suffering from a Lymphoid Malignancy such as Chronic Lymphocytic Leukemia, a disease in which B cell receptor stimulation is dysregulated or disease characterised by B lymphocyte proliferation/accumulation, comprising determining whether the patient's HS1 is in a phosphorylated form, and wherein an increased amount of the phosphorylated form relative to the unphosphorylated form indicates the presence of an agressive disease.

Described herein is a method of diagnosing a Lymphoid Malignancy such as lymphocytic leukemia or NHL, a disease in which B cell receptor stimulation is dysregulated or disease characterised by B lymphocyte proliferation/accumulation, in an individual comprising determining whether the patient's HS1 is in a phosphorylated form, and wherein an increased amount of the phosphorylated form relative to the unphosphorylated form indicates the presence of the disease.

Described herein is the use of a phosphorylated form of HS1 as a prognostic marker for a lymphoid malignancy (in particular CLL), a disease in which B cell receptor stimulation is dysregulated or disease characterised by B lymphocyte proliferation/accumulation, in combination with other already known markers that correlate with the diseases such as ZAP-70, CD38 and the levels of immunoglobulin V-gene mutation, as review in "Chronic Lymphocitic Leukemia" (Chiorazzi et al., N Engl J Med. 2005 Feb 24;352(8):804-15.).

In one embodiment the control is the amount of the phosphorylated form of HS1 in an individual who is not suffering from a lymphoid malignancy (as appropriate), a disease in which B cell receptor stimulation is dysregulated or disease characterised by B lymphocyte proliferation/accumulation.

In another preferred embodiment an appropriate control may be the relative amount between the phosphorylated and unphosphorylated form. Indeed, the inventors describe a prognostic method that is able to give an indication on poor versus good prognosis in CLL by measuring the relative amount between the two forms. Thus, patients with an increased relative amount of phosphorylated form are more easily exposed to an aggressive or poor prognosis.

Described herein is the use of an antagonist of HS1 phosphorylation for the preparation of a medicament for the treatment of a lymphoid malignancy, a disease in which B cell receptor stimulation is dysregulated or disease characterised by B lymphocyte proliferation/accumulation.

Described herein is the use of an antagonist of a phosphorylated HS1 for the preparation of a medicament for the treatment of a lymphoid malignancy, a disease in which B cell receptor stimulation is dysregulated or disease characterised by B lymphocyte proliferation/accumulation.

Described herein is the use of a means of dephosphorylating a phosphorylated HS1 for the preparation of a medicament for the treatment of a lymphoid malignancy, a disease in which B cell receptor stimulation is dysregulated or disease characterised by B lymphocyte proliferation/accumulation.

Described herein is a method of identifying an antagonist of HS1 phosphorylation, the method comprising: (a) providing a candidate molecule, (b) incubating the candidate molecule with HS1 in a phosphorylating environment, and (c) detecting whether phosphorylation of HS1 occurs.

By "phosphorylating environment" we mean an environment under which at least some phosphorylation will occur in the absence of an inhibitor of this process. The conditions under which such phosphorylation occurs are described later. In one method the candidate molecule is incubated with a B cell.

Described herein is a method of identifying an agonist of dephosphorylation, the method comprising: (a) providing a candidate molecule, (b) incubating the candidate molecule with phosphorylated HS1, and (c) detecting whether dephosphorylation of HS1 occurs.

In one embodiment the method further comprises isolating or synthesising a selected or identified molecule.

Described herein is an antagonist of HS1 phosphorylation identified according to the method of the present invention.

Described herein is an agonist of HS1 dephosphorylation identified according to the method of the present invention.

Described herein is a pharmaceutical composition comprising an antagonist or agonist of the present invention, together with a pharmaceutically acceptable excipient, diluent or carrier.

Described herein is use of the antagonist or agonist of the present invention in a method of treatment or diagnosis of a disease in an individual.

Described herein is a method of treatment of an individual suffering or likely to suffer from a disease comprising modulating (1) the phosphorylation of HS1, (2) the dephosphorylation of HS 1, and/or (3) the activity of phosphorylated HS 1.

As indicated above preferably the disease is a Lymphoid malignancy, a disease in which B cell receptor stimulation is dysregulated or disease characterised by B lymphocyte proliferation/accumulation.

### SUMMARY OF THE INVENTION

We have used a proteomic approach to identify molecules involved in the pathogenesis of chronic lymphocytic leukemia (CLL). We investigated 14 highly selected cases completely concordant for IgV_{H} mutational status (unmutated vs mutated), CD38 expression (positive vs negative) and clinical behavior (progressive vs stable), defined as either bad (7 cases) or good prognosis (7 cases). The two subsets differed for the expression of hematopoietic-lineage-cell-specific-protein-1 (HS1). In bad prognosis patients most HS1 protein was constitutively phosphorylated, whereas only a fraction was phosphorylated in good prognosis patients. This difference was investigated in a larger cohort of 26 unselected cases. The survival curve of all 40 cases analyzed revealed that patients with prevalently phosphorylated HS1 had a significantly shorter median survival time (p=0.024). As HS1 is a protein pivotal in the signal cascade triggered by the B-cell-receptor (BCR) stimulation, we studied its pattern of expression following BCR engagement. Normal mature B-cells stimulated by anti-IgM shifted the non- or less-phosphorylated form of HS1 toward the more phosphorylated form. Naive B-cells showed both HS1 forms while memory B-cells expressed mainly the phosphorylated fraction. These data indicate a central role for antigen stimulation in CLL and suggest a new therapeutic target for patients with aggressive disease. The present invention may also be applicable to other lymphoid malignancies and diseases in which B cell receptor stimulation is dysregulated or diseases characterised by B lymphocyte proliferation/accumulation.

Further particular and preferred aspects of the present invention are set out in the accompanying independent and dependent claims. Features of the dependent claims may be combined with features of the independent claims as appropriate, and in combinations other than those explicitly set out in the claims.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies : A Laboratory Manual : Portable Protocol NO. I by Edward Harlow, David Lane, Ed Harlow (1999, Cold Spring Harbor Laboratory Press, ISBN 0-87969-544-7); Antibodies : A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Fernandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3. Each of these general texts is herein incorporated by reference.

### BRIEF DESCRIPTION OF THE FIGURES

The present invention will be described further, by way of example only, with reference to preferred embodiments thereof as illustrated in the accompanying drawings, in which:

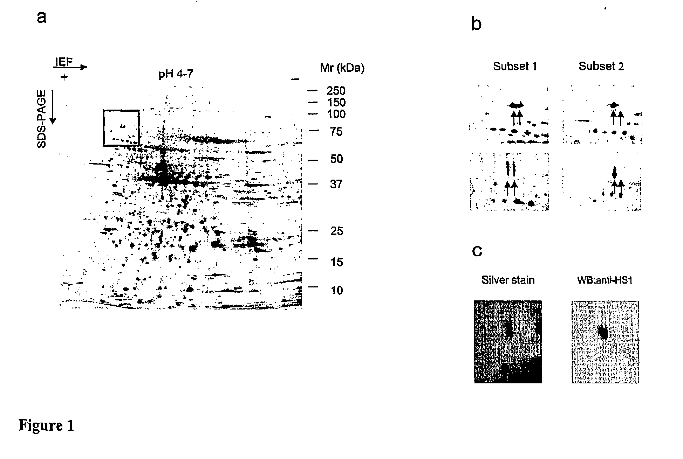

Figure 1 shows the results of 2-DE proteomic analysis of purified CLL cells. In more detail, (**a**) The square identifies two close spots with the same molecular mass (Mr) of 79 Kda and different isoeletric point (pI) of 4,83 and 4,86 respectively, which were identified by MALDI-TOF mass spectometry analysis as HS1 protein (see Results). (**b**) Both right and left spots are expressed in 2D-gels obtained from 2 representative *good prognosis* CLL patients (Subset 1) while only the more acidic one is present in 2D-gels obtained from 2 representative bad prognosis CLL patients (Subset 2). (c) Proteins obtained from 5 representative CLL patients were resolved on 2-DE that were either silver stained (left panel) or transferred onto nitrocellulose and incubated with an anti-HS1 antibody (right panel). One representative case is shown. The antibody hybridizes in an area corresponding to the spots of interest.

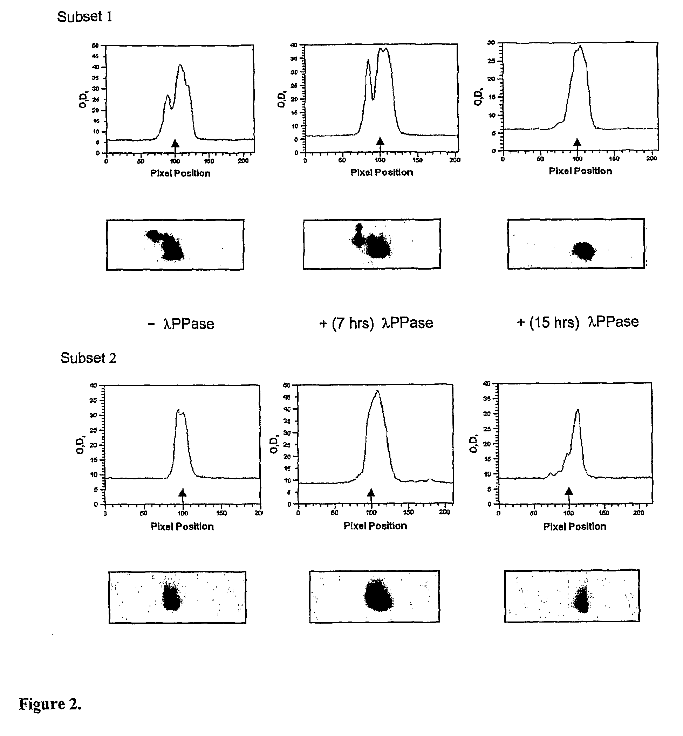

Figure 2 shows the results of 2-DE analysis illustrating that the more acidic spot is a phosphorylated form of HS1 protein. In more detail, cells from 4 CLL patients belonging to subset 1 and 2 were treated with λPPase and proteins resolved on 2 DE, transferred onto nitrocellulose and incubated with a specific anti-HS1 antibody at different time points.
One representative case is shown. The densitometric analysis indicates the progressive shift of the HS1 protein from a more to a less acidic form after 7 and 15 hours of incubation.

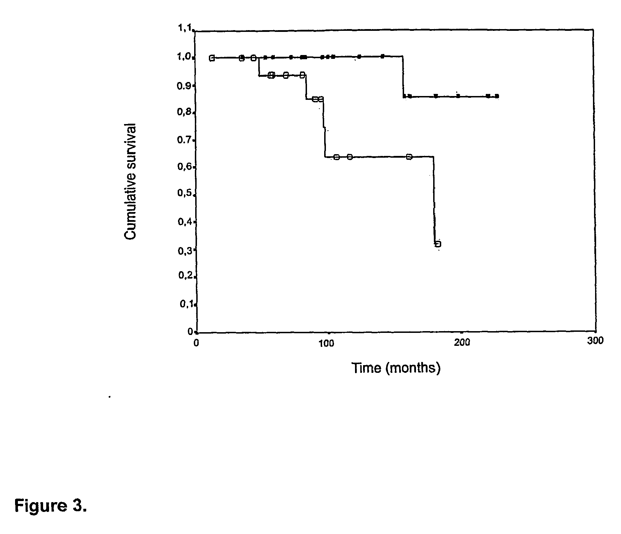

Figure 3 is a graph showing time against culmulative survival and illustrates that CLL patients with 1 HS1 spot have a shorter cumulative survival. In more detail, Kaplan-Meier curves show cumulative survival of CLL patients grouped according to HS1 expression pattern (1 single left spot vs 2 spots). Patients with 2 spots (black dots) have a significantly longer survival (median survival not reached) than those with only one (white dots) (median survival 184 months) (p=0.024).

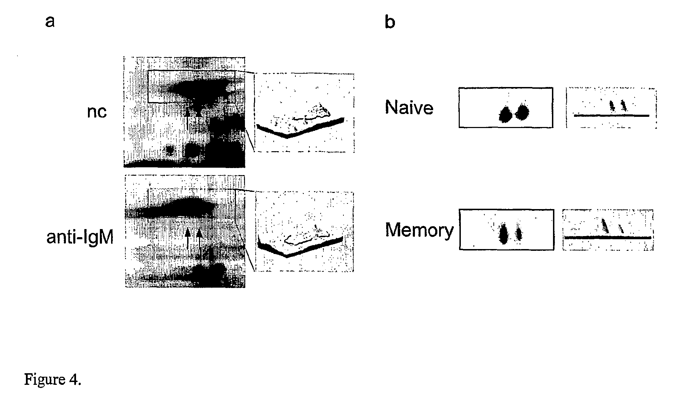

Figure 4 shows the HS1 pattern of expression in normal B lymphocytes. In more detail, (a) Purified tonsillar B cells were cultured in the presence or the absence of anti-IgM antibody for 2 minutes. Proteins from untreated and treated cells were resolved on 2-DE and revealed by silver staining. The arrows indicate the two HS1 spots and 3D-densitometric analysis (right panels) shows a shift from a less acidic to a more acidic form of HS1 protein, following BCR stimulation. (b) Proteins obtained from FACS-purified Naive and Memory tonsillar B cells were resolved on 2-DE and revealed by silver staining. The left panels identify the different HS1 forms. The 3D-densitometric analysis shows that both spots are expressed in similar amounts in Naive B cells while in Memory B cells the more acidic form is predominantly represented.

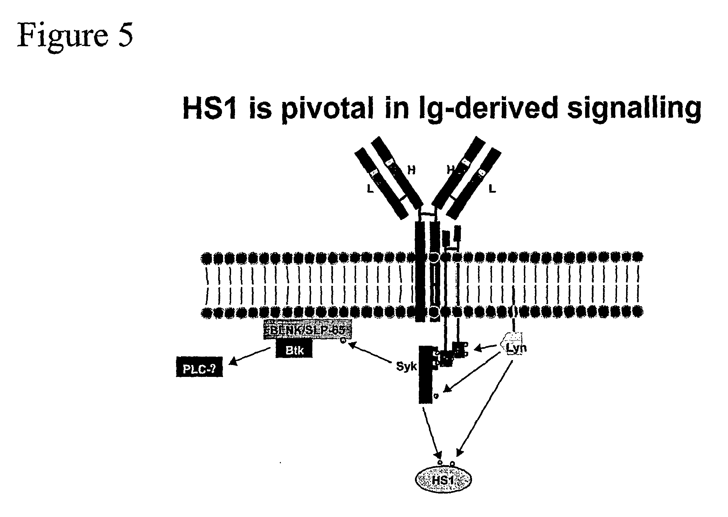

Figure 5 is a schematic illustrating the role of HS1 in Ig-derived signalling in a B lymphocyte.

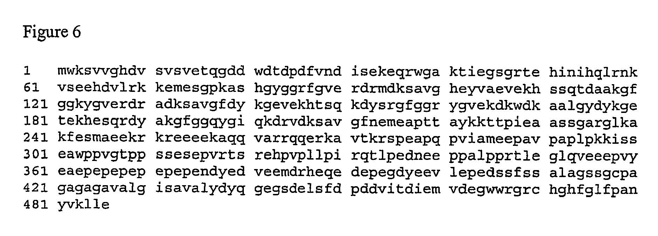

Figure 6 shows an amino acid sequence of HS1.

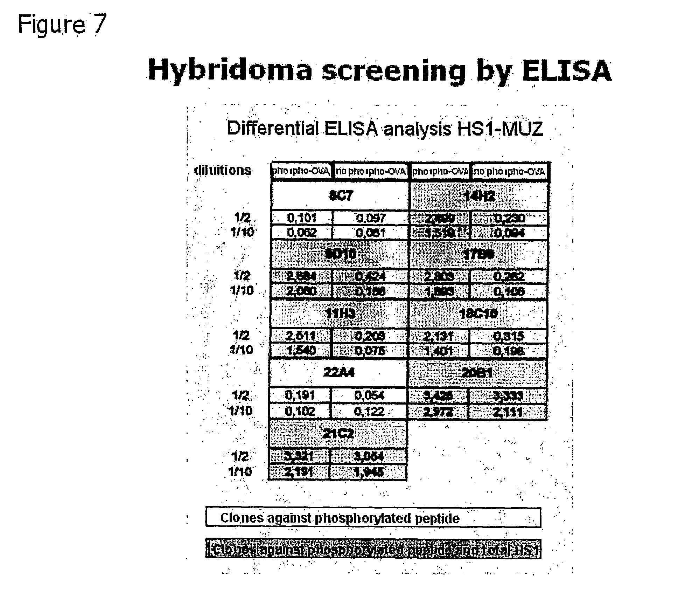

Figure 7 shows the results of hybridoma screening by ELISA from Example 13.

### DETAILED DESCRIPTION

CLL, the most common B cell tumor in adults, is characterized by the relentless expansion of monoclonal mature B lymphocytes. The biased use of certain immunoglobulin variable heavy chain (IgV_{H}) genes (1-5), the existence of similarities among Ig rearrangements (5-8) and the presence of IgV_{H} somatic mutations together with the typical expression of gene products associated with B-cell signaling and activation (9) raise the possibility that an antigenic drive may be instrumental in the malignant cell growth. The analysis of IgV_{H} somatic mutations, that track the clonal history to an in vivo activation of B-cell-receptor (BCR)-mediated activation, discriminates two distinct CLL subsets, one with somatically mutated (M) and one with unmutated (U) IgV_{H} genes (1, 4). The two subsets have a markedly different prognosis, U-CLL patients presenting a considerably shorter survival (2,10)

The presence of IgV_{H} somatic mutations suggests a role for BCR activation in the natural history of M-CLL, though these cases are typically unresponsive to BCR stimulation in nitro(11,12), similar to B cells that have undergone receptor desensitization, following chronic stimulation by antigen (13). Conversely, though expressing germline IgV_{H} genes, U-CLL strongly respond in vitro to anti-IgM stimulation, as shown by an increase in global tyrosine phosphorylation (11, 12) suggesting that they carry a more competent BCR, able to receive signals for maintenance or proliferation. In addition, CD38, a signaling molecule that possibly influences the outcome of BCR signaling once a specific surface expression threshold is reached (e.g. in the presence of IL-2) (14), also tends to be more represented in U-CLL (10). CD38 too has a significant prognostic value which is independent from IgV_{H} somatic mutations (15). Interestingly, a strong correlation between CD38 expression and responsiveness to signaling via sIgM (16, 17) has been reported and appears to be enhanced by the expression of the signaling molecule ZAP-70 (11, 18). The expression of ZAP-70(19), a kinase that shares functions with Syk, is also associated with the unmutated IgV_{H} status (20-23).

The markedly different response to the stimulation of BCR in the cells from CLL subsets characterized by a distinct clinical behaviour leads to ask whether the stimulation of BCR has a role in the onset or is also involved in the maintenance of the disease and to what extent these two possible scenarios may apply to the different subsets of patients. We have used a 2-dimensional electrophoresis (2-DE) proteomic approach to study the cells from 40 CLL cases, characterized on the basis of their biological and clinical features. By 2-DE and mass spectrometry (MS) analysis we have found that the hematopoietic lineage cell-specific protein 1 (HS 1), an intracellular protein which is pivotal in the signal cascade triggered by the BCR stimulation, is differentially expressed and shows distinct features in different subsets of patients.

The use of gene expression profiling has shown that CLL cases, irrespective of Ig mutational status, are characterized by a common gene expression "signature" (23, 29), suggesting that all CLL share a common mechanism of transformation and/or cell of origin. That notwithstanding, CLL is heterogeneous both at clinical and biological level. CLL patients can be grouped into different subsets on the basis of biological features that have been shown to predict the clinical behaviour (aggressive vs indolent). A number of these features are related to differences in the cell capacity to respond to signals originated from the microenvironment. A prominent example is the presence of somatic hypermutations of IgV_{H} genes which reveals the stimulation of BCR (2,10).

In this work we have first analyzed a number of CLL cases purposefully selected at the two extremes of a Gaussian curve to minimize interpatient variations. The patients were grouped into two subsets (good prognosis and bad prognosis) clustered on the basis of the complete concordance of biological factors (IgV_{H} mutational status and CD38 expression) and clinical outcome (stable or progressive disease). This approach allowed to show that the features of the protein HS1 significantly differ in the two groups as most HS1 protein detected in the cells from bad prognosis patients is constitutively phosphorylated, whereas only a minor fraction is phosphorylated in the cells from the good prognosis subset (Fig. 1). This finding was confirmed by adding to the study a larger unselected series of 26 cases. Overall the proteomic pattern of HS1 maintained a significant correlation with both the IgV_{H} mutational status and the expression of CD38. The rather unexpected lack of correlation with the expression of Zap-70 may simply reflect the size of the cohort of patients studied. Of interest, when the 40 CLL patients studied were pooled together and their survival curves were related to the profile of HS1, the cases presenting a single left HS1 spot on the 2-D gels - and thus showing a prevalently phosphorylated form - resulted to have a median survival significantly shorter than those presenting two spots, i.e. a prevalently unphosphorylated HS1 form (Fig.3).

HS1 is a 79 kD intracellular protein expressed exclusively in cells of lymphohemopoietic origin. In B and T lymphocytes it plays a relevant role in signal transduction through surface antigen receptors (24, 30). In B lymphocytes it undergoes rapid phosphorylation at several tyrosine residues following BCR stimulation (24-26) and is an important substrate of protein tyrosin kinases such as Syk and Src family proteins including Lyn, Fgr, Fyn and Lck. Targeted-mice have revealed that HS1 has a central role in B cell responsiveness, as HS1-deficient mice have a defective antigen-induced clonal expansion and deletion (31).

We have found that both in vitro and in vivo stimulation of normal B cells through the BCR is sufficient to phosphorylate HS1 protein. This finding is well in keeping with the observation that in normal T lymphocytes (32) costimulation activates the same signaling molecules triggered by the antigen receptor stimulation, but does not lead to phosphorylation of HS1. This evidence indicates that the phosphorylation of HS1 is induced by the stimulation of antigen receptor and suggests that in B cells HS 1 phosphorylation can be considered a signature of BCR engagement. Therefore, our findings that in malignant CLL cells from the bad prognosis group most of HS1 protein is in the phosphorylated form suggest the presence of an ongoing stimulation likely triggered by a recent/persistent BCR-mediated activation. This possibility is supported by the fact that in vitro anti-IgM stimulation may induce phosphorylation of Syk in bad prognosis patients [see also (11, 12)]. These data reinforce the concept emerged from the study of Ig gene rearrangement features(5, 33, 34) that the stimulation of BCR appears to have a relevant role in cases with a more aggressive behavior. Whilst not wishing to be bound by any theory, taken together these observations indicate that circulating U-CLL cells maintain the full capacity to respond to a still unknown triggering antigen to which they may be chronically exposed. The implication is that in bad prognosis CLL the stimulation of BCR appears to be relevant not only for the onset but also for the maintenance and progression of the disease.

In contrast good prognosis cases tend to have HS1 in the non-phosphorylated form and are unable to phopsphorylate Syk upon anti-IgM stimulation. These features are in keeping with the notion that mutated cases show, at least in vitro, a characteristic BCR unresponsiveness (11, 12). This is generally considered a sign of an anergic state, likely induced after an antigen-like stimulation (35). This suggests that in good prognosis patients antigen stimulation is an event that has occurred at the onset of the disease, but that an ongoing BCR stimulation is not necessary for its maintenance. Rather other factors originating from the microenvironment (36) such as stimulating cytokines might help maintaining the stimulated clone.

In both instances the stimulus which sustains the BCR activation in patients may be a foreign or an auto-antigen. Considering the skewing of Ig gene usage (1, 3, 4) and the fact that a high proportion of CLL B cells display natural autoAb activity (37) it follows that the most likely candidate is an autoantigen.

Thus, it appears that antigen stimulation may have a central role in the natural history of CLL and especially of poor prognosis U-CLL. The very fact that bad prognosis CLL is characterized by the prominent expression of the phosphorylated form of HS1 indicates a potential for defining new diagnostic or prognostic tools that may be applied to patients at diagnosis in order to identify cases that are likely to run a progressive course. More importantly it suggests a new molecular target for the treatment of patients who present an aggressive disease.

The role of HS1 in Ig-derived signalling is illustrated schematically in Figure 5.

### DISEASES

The present invention relates to the diagnosis or prognosis and treatment of Lymphoid Malignancies such as Chronic Lymphocytic Leukemia.

Chronic lymphocytic leucemia is an indolent Lymphoid Malignancy chracterized by the relentless accumulation in the peripheral blood of small, resting B lymhpocytes expressing CD5 on the cell surface. It arises from the bone marrow and infiltrates secondary lymphoid tissues as lymph nodes and spleen. CLL is characterized by lymphocytosis, anemia and thrombocytopenia and typically by immunodeficiency with progressive hypogammaglobulinemia as well as autoimmune manifestations caused by polyclonal autoantibodies.

Lymphoid Malignancies arise from cells of the immune system at different stages of differentiation and include Hodgkin's disease (HD), Non-Hodgkin lymphomas (NHL) and other entities, as recently classified by the World Health Organization. Most lymphoid malignancies are of B cell origin (89-90%). It will be appreciated that the present invention may have applicability to such lymphoid malignancies and particularly to those of B cell origin, both indolent and aggressive.

It will be appreciated that the present invention is applicable to other diseases which involve or are characterised by B cell receptor stimulation dysregulation or diseases characterised by B lymphocyte proliferation/accumulation. These include immune disorders.

As indicated above, the present invention is useful in treating immune disorders such as autoimmune diseases or graft rejection such as allograft rejection.

Examples of disorders that may be treated include a group commonly called autoimmune diseases. The spectrum of autoimmune disorders ranges from organ specific diseases (such as thyroiditis, insulitis, multiple sclerosis, iridocyclitis, uveitis, orchitis, hepatitis, Addison's disease, myasthenia gravis) to systemic illnesses such as rheumatoid arthritis or lupus erythematosus. Other disorders include immune hyperreactivity, such as allergic reactions.

In more detail: Organ-specific autoimmune diseases include multiple sclerosis, insulin dependent diabetes mellitus, several forms of anemia (aplastic, hemolytic), autoimmune hepatitis, thyroiditis, insulitis, iridocyclitis, skleritis, uveitis, orchitis, myasthenia gravis, idiopathic thrombocytopenic purpura, inflammatory bowel diseases (Crohn's disease, ulcerative colitis).

Systemic autoimmune diseases include: rheumatoid arthritis, juvenile arthritis, scleroderma and systemic sclerosis, sjogren's syndrom, undifferentiated connective tissue syndrome, antiphospholipid syndrome, different forms of vasculitis (polyarteritis nodosa, allergic granulomatosis and angiitis, Wegner's granulomatosis, Kawasaki disease, hypersensitivity vasculitis, Henoch-Schoenlein purpura, Behcet's Syndrome, Takayasu arteritis, Giant cell arteritis, Thrombangiitis obliterans), lupus erythematosus, polymyalgia rheumatica, essentiell (mixed) cryoglobulinemia, Psoriasis vulgaris and psoriatic arthritis, diffus fasciitis with or without eosinophilia, polymyositis and other idiopathic inflammatory myopathies, relapsing panniculitis, relapsing polychondritis, lymphomatoid granulomatosis, erythema nodosum, ankylosing spondylitis, Reiter's syndrome, different forms of inflammatory dermatitis.

A more extensive list of disorders includes: unwanted immune reactions and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimoorchitis, infertility, orchidal trauma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery or organ, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

### HS1 PHOSPHORYLATION USED AS PROGNOSTIC TOOLS

The following describes HS1 phosphorylation sites that may be used in accordance with the present invention to determine whether phosphorylation of HS1 has occurred. HS1 undergoes a process of sequential phosphorylation both in vitro and in vivo, which is synergistically mediated by Syk and Src family protein-tyrosine kinases. Tyrosine 222 has been identified as the HS1 residue phosphorylated by the Src family protein kinases. Tyrosine 397 (and possible tyrosine 398) have been indentified as the HS1 residue phosphorylated by Syk. In one aspect of the present invention the existence or extent of phosphorylation at these, or other yet unidentified sites can be used as a diagnostic or prognostic tool.

Without wishing to be bound by any theory, we believe HS1 undergoes phosphorylation on two tyrosine residues (Y378 and Y397) followed by successive phosphorylation at another site (Y222). Thus the existence, or extent on a population, of phosphorylation at tyrosine residues 378, 397 and/or 222 can be used as a diagnostic or prognostic tool, or in the screening methods of the present invention.

The presence of phosphorylation may be determined using any suitable means know in the art and as described in the accompanying Examples. Non-limiting examples of such methods include:
HS1 Detection:
   - Multisample horizontal electrofocusing
   - Single-sample 2-D electrohoresis (2-DE)
Phosphorylated-HS1 Detection:
   - Western Blot
   - FACS
   - Confocal microscopy

For detecting phosphorylation and for use in diagnostic purposes, antibodies may be useful. For example, anti-Lyn and anti-Syk antibodies raised against protein residues 44-63 and 257-352 respectively may be useful. Phosphopeptides-specific antibodies HS1 (P-Tyr397) and HS1(P-Tyr222) and HS1 (P-Tyr378) may be developed. In particular embodiments the antibodies may be generated against phosphopeptides corresponding to the following sequences of human HS1, Cys-APTTA(P)YKKTTT (Aa 217-226) and Cys-PEGD(P)YEEVLE (Aa 393-402) and Cys-PEND(P)YEDVEEMDR (Aa 374-386). These antibodies may be generated, for example, in New Zealand rabbits or using other standard methods as described below.

### THERAPY

The present invention also relates to methods of treating lymphoid maligancies and other diseases in which B cell receptor stimulation is dysregulated or other diseases characterised by B lymphocyte proliferation.

Therapy may be achieved by blocking phosphorylation of HS1, or by encouraging dephosphorylation thereof. Means of achieving therapy include the use of inhibitors of phosphorylation, inhibitors of phophorylated HS1 or compounds which achieve dephosphorylation of HS1.

Inhibition of phosphorylation may occur at any point in the phosphorylation pathway.

In more detail, thrombin-stimulation of platelets triggers the Tyr-phosphorylation of several signalling proteins. HS1 undergoes a transient Tyr-phosphorylation in human platelets stimulated with thrombin.

CLL B cells consistently express mRNA for VEGF receptor 1 (VEGFR1), thrombin receptor, endoglin, and angiopoietin. Further analysis of VEGFR expression by RT-PCR revealed that CLL B cells expressed both VEGFR1 mRNA and VEGFR2 mRNA.
(http://veille-
srv.inist.fr/bin/dilib/AppliHuman/doc.fibres.cgi?/applis/veille/home/apache/users/genomiq ue/Human/Server/EN.fibres.Genes.wsh+013658. Thus in one aspect the present invention envisages the use of an antagonist of thrombin or more generally an antagonist of thrombin-induced actin assembly. An example of a compound which inhibits thrombin-induced actin assembly is cytochalasin D. It has been established that thrombin-stimulation of platelets triggers a rapid rearrangement of the actin cytoskeleton and that Src and Syk tyrosine kinases are implicated in this process. HS1 contains an F-actin binding domain at the N-terminus, which has been suggested to interact with the actin fragments and regulate their assembly. Thus, antagonists directed to this F-actin binding domain may have activity in the present invention.

The HS1 protein is synergistically phosphorylated by Syk and Lyn tyrosine kinases according to a sequential phosphorylation mechanism. Thus, in another embodiment the present invention relates to the use of antagonists of Syk and Lyn.

Syk acts as a primary kinase which phosphorylates HS1 at Tyr397. Upon docking to Syk-phosphorylated HS 1, Lyn catalyses the secondary phophorylation of the protein at Tyr222. Inhibition of other tyrosine kinases belonging to the Syk/ZAP70 family may similarly be useful in the present invention, as the concerted action of the non-receptor tyrosine kinases belonging to this family is a general theme in hematopoietic cell signal transduction. Examples of inhibitors of the Syk/ZAP70 family include picaeatannol (3,4,3',5'-tetrahydroxy-trans-stilbene), ER-27319 (3,4-dimethyl-10-(3-aminopropyl)-9-acridone oxalate), acridone-related compounds, Lys-Leu-Ile-Leu-Phe-Leu-Leu-Leu peptide and peptides containing the Lys-Leu-Ile-Leu-Phe-Leu-Leu-Leu motif. Other examples of compounds which are useful as inhibitors of Syk are given in WO2004/058749. In this publication the inhibitors are mentioned as being useful in the treatment of chronic myelogenous leukemia (CML), acute myeloid leukemia (ACL) and acute promyelocytic leukemia (APL), but there is no specific mention of their use in the treatment of CLL.

Lyn belongs to the Src family which consists of at least nine non-receptor tyrosine kinases - Lyn and Src, Fyn, Fgr, Lck, Hck, Yes, Yrk and Blk. Direct association of such Src-related tyrosine kinases with HS1 has been described. Thus it is envisaged that inhibitors of Src-related tyrosine kinases other than Lyn may be useful.

Examples of Lyn antagonists which find use in the present invention include PP2 and SU6656. Other inhbitors of the Src family include SU101 and CGP57418B [Broadbridge RJ et al, The Src homology-2 domains (SH2 domains) of the protein tyrosine kinase p561ck: structure, mechanism and drug design, Current Drug Targets (2000) 1(4):365-86]. Epigallocatechin-3-gallate (EGCG) is another inhibitor of Lyn, and although this green tea consitituent is known to act as a cancer-preventative agent because of its ability to induce apoptosis in cancer cells by caspase activation, its use in accordance with the present invention has not previously been disclosed.

Syk phosphorylates Tyr378 and Tyr397 of HS1 and antagonists, such as small molecules and in particular peptides, directed to these Tyrosine residues, as well as Tyrosine 222, and the SH2 domain will find utility in the present invention (for therapy).

Thus according to a first aspect of the present invention, we provide an antagonist of a tyrosine kinase implicated in the phosphorylation of HS1.

Dephosphorylation may be achieved in a number of ways, for example:

### A) Activating HS1 specific phosphatases.

In literature, it has been shown that phosphorylated HS1 undergoes dephosphorylation and thus there may be a specific HS 1 dephosphorylase which could be employed in the present invention.

Taken from Brunati et al., Mar 2005, J. Bio. Chem *:*

*"After Lyn phosphorylation, HS1 would become substrate of protein tyrosine phosphatase(s), which would rapidly reduce the Tyr-phosphorylation extent of the protein. This hypothesis is supported by the experiments performed with the tyrosine phosphatase inhibitor pervanadate (**Fig. 1**) and by the finding that HS1 only primarily phosphorylated does not undergo dephosphorylation (Figs. 2C and* *3**)*. *Therefore, when Lyn is inhibited, Syk-phosphorylated HS1 is recruited by Lyn SH2 domain and can not be dephosphorylated by tyrosine phosphatase(s).*

*Indeed, Syk acts as primary kinase which phosphorylated HS1 at Tyr397 and that Syk-phosphorylation is required for HS1 interaction with Lyn SH2-domain. Upon docking to Syk-phosphorylated HS1, Lyn catalyzes the secondary phosphorylation of the protein at Tyr222. Once the secondary Tyr-phosphorylation of HS1 is accomplished the protein dissociates from Lyn and undergoes a dephosphorylation process".*

### B) Inhibiting Ser/Thr phosphorylation

The dephosphorylation process is hindered by HS1 phosphorylation Ser/Thr residues mediated by protein kinase CK2, as it becomes resistant to degradation.

*"*Protein kinase CK2 inhibitors 4, 5, 6, 7-tetrabromobenzotriazole (TBB) induces apoptosis and caspase-dependent degradation of haematopoietic lineage cell-specific protein 1 (HS1) in Jurkat cells. Ruzzene M, Penzo D, Pinna LA. Biochem J. 2002 May 15;364(pt 1):41-7*"*

Incubation of Jurkat cells with 4,5,6,7-tetrabromobenzotriazole (TBB), a specific inhibitor of protein kinase CK2, induces dose-and time-dependent apoptosis as judged by several criteria. TBB-promoted apoptosis is preceded by inhibition of Ser/Thr phosphorylation of haematopoietic lineage cell-specific protein 1(HS1) and is accompanied by caspase-dependent fragmentation of the same protein.

Moreover, in vitro experiments show that HS1, once phosphorylated by CK2, becomes refractory to cleavage by caspase-3. These findings, in conjunction with similar data in the literature concerning two other CK2 protein substrates, Bid and Max, suggest that CK2 may play a general anti-apoptotic role through the generation of phosphorylated sites conferring resistance to caspase cleavage.

Thus we could envisage using inhibitors of CK2 activity. A specific inhibitor of CK2 has already been described in the same paper mentioned earlier.

The methods and compositions described here are generally concerned with inhibitors of protein activity, preferably enzyme activity. In particular, we describe antagonists of protein activity, specifically, antagonists of tyrosine kinase. Such antagonists are capable of modulating, including preferably inhibiting, down-regulating or abolishing the phosphorylating activity of the tyrosine kinase.

In one embodiment, the antagonist of tyrosine kinase comprises a peptide, or an entity comprising a peptide (for example, a peptide associated with another entity, which may itself be a peptide). The term "peptide", as used in this document, refers generally to a polymer of amino acids. It includes, for example, di-peptides, tri-peptides, oligopeptides and polypeptides.

Preferably, the peptide comprises "natural" amino acids, that is to say, amino acids as they exist in peptides in nature. However, modified versions of amino acids, which may be derivatised to change one or more activities or properties, for example, water solubility, are also included.

The peptide antagonist may be made by biochemical methods, for example, protein digestion of a native tyrosine kinase, or preferably by recombinant DNA methods as known in the art. Accordingly, it will be understood that the antagonists specifically include recombinant peptides. The peptide antagonists also include homologous sequences obtained from any source, for example related viral/bacterial proteins, cellular homologues and synthetic peptides, as well as variants or derivatives thereof. Thus peptide antagonists also include sequences from homologues of tyrosine kinases from other species including other microorganisms. Furthermore, homologues from higher animals such as mammals (e.g. mice, rats or rabbits), especially primates, more especially humans are also included.

The antagonists of tyrosine kinase described here in one embodiment are based on tyrosine kinase, and preferably comprise at least a portion of a tyrosine kinase. Human sequences are preferred.

Antagonists of tyrosine kinase may also be derived from HS1 sequences, so that they comprise at least a portion of HS 1. For example, HS1 and / or tyrosine kinase sequences from rat, mouse or human may be used.

The peptide isoform specific antagonists of tyrosine kinase may comprise other sequences, for example sequences from other proteins or polypeptides. In particular, they may comprise sequences for expression, for tagging, for solubility, and for membrane penetration. Where the antagonists of tyrosine kinase comprise more than one peptide or more than one sequence, the peptides or sequences need not be contiguous, and in particular, need not be joined by peptide bonds. For example, they may be conjugated to each other, by use of crosslinkers as known in the art. However, they are preferably provided as fusion proteins, for example, a fusion protein comprising GST.

The antagonists of phosphorylation may be of any suitable length, for example, 50, 45, 40, 35, 30, 25, 24, 23, 22, 21, 20, 19, 18, 17, 16, 15, 14, 13, 12, 11, 10 or 9 or fewer residues. Preferably, they comprise or consist of between 21 to 10 amino acid residues, more preferably 21, 20 or 13 amino acid residues.

Preferably, the antagonist of tyrosine kinase comprises a molecule capable of modulating an interaction between an SH2 containing polypeptide, such as HS1, and a tyrosine kinase.

### ANTAGONISTS/INHIBITORS

The methods and compositions described here rely, in some embodiments, on blocking phosphorylation of HS1 such as by blocking an activity of a tyrosine kinase, for example Syk or Lyn. Agents which are capable of decreasing or inhibiting phosphorylation activity are referred to as antagonists or inhibitors of that activity.

The term "antagonist", as used in the art, is generally taken to refer to a compound which binds to an enzyme and inhibits the activity of the enzyme. The term as used here, however, is intended to refer broadly to any agent which inhibits the activity of a molecule, not necessarily by binding to it. Accordingly, as used generally, it includes agents which affect phosphorylation of HS 1, for example the expression of a tyrosine kinase, for example Syk or Lyn, or the expression of modulators of the activity of the tyrosine kinase.

The antagonist may bind to and compete for one or more sites on the relevant molecule, for example, HS1, or a tyrosine kinase, for example Syk or Lyn, preferably, a catalytic site or a binding site of the tyrosine kinase. The antagonist of the kinase preferably interferes with, or prevents, the binding of HS1 to the kinase. Preferably, it competes for an HS1 binding region of the kinase.

Preferably, such binding blocks the interaction between the molecule and another entity (for example, the interaction between the tyrosine kinase and HS1). However, the antagonist need not necessarily bind directly to a catalytic or binding site, and may bind for example to an adjacent site, for example, an adjacent site in the kinase polypeptide, or even another protein (for example, a protein which is complexed with the enzyme) or other entity on or in the cell, so long as its binding reduces the activity of the enzyme or molecule or molecules in question.

Where antagonists of a enzyme such as a tyrosine kinase enzyme are concerned, an antagonist may include a substrate of the enzyme, or a fragment of this which is capable of binding to the enzyme. An example is HS 1. In addition, whole or fragments of a substrate generated natively or by peptide synthesis may be used to compete with the substrate for binding sites on the enzyme. The antagonist may also include a peptide or other small molecule which is capable of interfering with the binding interaction. Other examples of antagonists are set forth in greater detail below, and will also be apparent to the skilled person.

Blocking the activity of a tyrosine kinase may also be achieved by reducing the level of expression of the kinase in the cell. For example, the cell may be treated with antisense compounds, for example oligonucleotides having sequences specific to the tyrosine kinase mRNA.

As used herein, in general, the term "antagonist" includes but is not limited to agents such as an atom or molecule, wherein a molecule may be inorganic or organic, a biological effector molecule and/or a nucleic acid encoding an agent such as a biological effector molecule, a protein, a polypeptide, a peptide, a nucleic acid, a peptide nucleic acid (PNA), a virus, a virus-like particle, a nucleotide, a ribonucleotide, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a fatty acid and a carbohydrate. An agent may be in solution or in suspension (e.g., in crystalline, colloidal or other particulate form). The agent may be in the form of a monomer, dimer, oligomer, etc, or otherwise in a complex.

The terms "antagonist" and "agent" are also intended to include, a protein, polypeptide or peptide including, but not limited to, a structural protein, an enzyme, a cytokine (such as an interferon and/or an interleukin) an antibiotic, a polyclonal or monoclonal antibody, or an effective part thereof, such as an Fv fragment, which antibody or part thereof may be natural, synthetic or humanised, a peptide hormone, a receptor, a signalling molecule or other protein; a nucleic acid, as defined below, including, but not limited to, an oligonucleotide or modified oligonucleotide, an antisense oligonucleotide or modified antisense oligonucleotide, cDNA, genomic DNA, an artificial or natural chromosome (e.g. a yeast artificial chromosome) or a part thereof, RNA, including mRNA, tRNA, rRNA or a ribozyme, or a peptide nucleic acid (PNA); a virus or virus-like particles; a nucleotide or ribonucleotide or synthetic analogue thereof, which may be modified or unmodified; an amino acid or analogue thereof, which may be modified or unmodified; a non-peptide (e.g., steroid) hormone; a proteoglycan; a lipid; or a carbohydrate. Small molecules, including inorganic and organic chemicals, which bind to and occupy the active site of the polypeptide thereby making the catalytic site inaccessible to substrate such that normal biological activity is prevented, are also included. Examples of small molecules include but are not limited to small peptides or peptide-like molecules.

### MODIFIED CELLS

According to the methods and compositions described here, modulation of phosphorylation of HS1 in a cell improves cell viability of a population. Thus reduction of expression of a tyrosine kinase may lead to improved cell viability. However, it will be appreciated that methods of regulation of any of these genes, including use of modulator entities such as agonists and antagonists, may be employed in addition to, or as an alternative to, modulation of polypeptide expression.

Cells in which the expression of any one or more of these genes are modulated are referred to for convenience as "modified" cells - although it will be appreciated that these may not be physically modified themselves, but may be descendants of cells which have been modified.

The relevant cells may be modified by targeting relevant genes by any means known in the art.

One possible approach is to express anti-sense constructs directed against tyrosine kinases to inhibit gene function and prevent the expression of the relevant polypeptide. Another approach is to use non-functional variants of polypeptides that compete with the endogenous gene product resulting in inhibition of function. Alternatively, compounds identified by the assays described above as binding to a polypeptide may be administered to cells to prevent the function of that polypeptide. This may be performed, for example, by means of recombinant DNA technology or by direct administration of the compounds. Suitable antibodies may also be used as agents.

Alternatively, double-stranded (ds) RNA is a powerful way of interfering with gene expression in a range of organisms that has recently been shown to be successful in mammals (Wianny and Zernicka-Goetz, 2000, Nat Cell Biol 2000, 2, 70-75). Double stranded RNA corresponding to the sequence of a tyrosine kinase polynucleotide can be introduced into or expressed in cells or cell lines to enhance cell viability.

In particular, we describe modification by the use of single interfering RNAs (siRNAs) as well as the use of dominant negative mutants where reduction in expression is desired. We further describe the use of vectors which enable over-expression of a relevant sequence for increasing expression of relevant genes. The modification may be transient, or it may be permanent. Thus, we provide for cell lines which comprise cells with genomic and transmittable modifications in tyrosine kinases.

We further provide for transgenic animals whose cells comprise down-regulated expression of tyrosine kinases.

Preferably, cell viability is gauged by quantitating a viable cell density of a population of cells which have been modified. Preferably, the modified cells maintain a higher cell viability, compared to cells which have not been modified (e.g., a control population). Cell viability is preferably measured as the percentage of cells in the relevant cell population which are viable.

In a preferred embodiment, cell viability is determined by a "Trypan blue viability exclusion assay". This assay is commonly used for cell viability determination in the field of cell culture.

Preferably, the modified cells have at least 5%, preferably 10% or more, more preferably 15%, 20%, 30%, 40%, 50% or more viable cells compared to a control population. Alternatively, or in addition, the modified cells maintain cell viability for a longer period of time compared to cells which have not been modified. For example, modified cells are able to maintain a certain percentage cell viability (e.g., 95%) for a longer period compared to control cells.

Preferably, modified cells have extended cell viability by at least 1 hour, more preferably at least 6 hours, most preferably at least 12 hours or more, e.g., at least 24 hours, at least 36 hours or at least 48 hours, compared to control cells. In highly preferred embodiments, modified cells have extended viability by at least 24 hours before viability begins to drop below 95%, compared to control cells.

### SMALL MOLECULE ANTAGONISTS

In addition to peptides, small molecules may be used to specifically inhibit phosphorylation. We therefore disclose small molecule α inhibitors, as well as assays for screening for these. Small molecule antagonists of tyrosine kinase activity are screened by detecting modulation, preferably down regulation, of binding or other activity.

By "down-regulation" we include any negative effect on the behaviour being studied; this may be total, or partial. Thus, where binding is being detected, candidate antagonists are capable of reducing, ameliorating, or abolishing the binding between two entities. Preferably, the down-regulation of binding (or any other activity) achieved by the candidate molecule is at least 10%, preferably at least 20%, preferably at least 30%, preferably at least 40%, preferably at least 50%, preferably at least 60%, preferably at least 70%, preferably at least 80%, preferably at least 90%, or more compared to binding (or which ever activity) in the absence of the candidate molecule. Thus, a candidate molecule suitable for use as an antagonist is one which is capable of reducing by 10% more the binding or other activity.

The assays may comprise binding assays. In general, such binding assays involve exposing a tyrosine kinase or HS1 polypeptide, nucleic acid, or a fragment, homologue, variant or derivative thereof to a candidate molecule and detecting an interaction or binding between the tyrosine kinase or HS1, respectively, polypeptide, nucleic acid, or a fragment, homologue, variant or derivative thereof and the candidate molecule. The binding assay may be conducted *in vitro,* or *in vivo.* They may be conducted in a test tube, comprising substantially only the components mentioned, or they may be conducted using cell free extracts or more or less purified components. The assays may be conducted within a cell, tissue, organ or organism.

In some embodiments, the assays are conducted on whole organisms rather than cells. Preferably, the organism is one which suffers from a disease as disclosed in this document, or exhibits one or more symptoms of such a disease. The nature etc of candidate molecules are discussed in further detail below, and in particular, they may be in the form of a library, also discussed below.

The candidate molecules are contacted with the test protein or proteins, and an effect detected. In the simplest assay, for example, the candidate molecule is contacted with a tyrosine kinase or HS1, and binding to the tyrosine kinase or HS1 respectively tested. An assay may also involve contacting the candidate molecule with a peptide comprising a relevant portion of a tyrosine kinase or HS 1. Binding of the candidate molecule of the candidate molecule to the peptide is detected.

Another binding assay useful in identifying antagonists of tyrosine kinase involves detecting modulation of binding between a peptide comprising a HS1 binding portion of the tyrosine kinase, and HS1 protein, in the presence of a candidate molecule. Candidate molecules which modulate binding between the peptide and HS1, preferably down-regulate this binding, are chosen.

One type of assay for identifying substances that bind to a polypeptide involves contacting a polypeptide, which is immobilised on a solid support, with a non-immobilised candidate substance determining whether and/or to what extent the polypeptide and candidate substance bind to each other. Alternatively, the candidate substance may be immobilised and the polypeptide non-immobilised. This may be used to detect substances capable of binding to tyrosine kinase or HS1, or fragments, homologues, variants or derivatives thereof.

In a preferred assay method, the polypeptide is immobilised on beads such as agarose beads. Typically this is achieved by expressing the tyrosine kinase or HS1, or a fragment, homologue, variant or derivative thereof as a GST-fusion protein in bacteria, yeast or higher eukaryotic cell lines and purifying the GST-fusion protein from crude cell extracts using glutathione-agarose beads (Smith and Johnson, 1988). As a control, binding of the candidate substance, which is not a GST-fusion protein, to the immobilised polypeptide is determined in the absence of the polypeptide. The binding of the candidate substance to the immobilised polypeptide is then determined. This type of assay is known in the art as a GST pull down assay. Again, the candidate substance may be immobilised and the polypeptide non-immobilised.

It is also possible to perform this type of assay using different affinity purification systems for immobilising one of the components, for example Ni-NTA agarose and histidine-tagged components.

Binding of the tyrosine kinase or HS1, or a fragment, homologue, variant or derivative thereof to the candidate substance may be determined by a variety of methods well-known in the art. For example, the non-immobilised component may be labeled (with for example, a radioactive label, an epitope tag or an enzyme-antibody conjugate). Alternatively, binding may be determined by immunological detection techniques. For example, the reaction mixture can be Western blotted and the blot probed with an antibody that detects the non-immobilised component. ELISA techniques may also be used.

Candidate substances are typically added to a final concentration of from 1 to 1000 nmol/ml, more preferably from 1 to 100 nmol/ml. In the case of antibodies, the final concentration used is typically from 100 to 500 µg/ml, more preferably from 200 to 300 µg/ml.

Candidate molecules may be isolated or synthesised by means known in the art, and if needed used for further study.

### CANDIDATE MOLECULES

Suitable candidate molecules for use in the above assays include peptides, especially of from about 5 to 30 or 10 to 25 amino acids in size. Peptides from panels of peptides comprising random sequences or sequences which have been varied consistently to provide a maximally diverse panel of peptides may be used.

Furthermore, combinatorial libraries, peptide and peptide mimetics, defined chemical entities, oligonucleotides, and natural product libraries may be screened for activity. The candidate molecules may be used in an initial screen in batches of, for example 10 types of molecules per reaction, and the molecules of those batches which show enhancement or reduction of the activity being assayed tested individually.

### LIBRARIES

Libraries of candidate molecules, such as libraries of polypeptides or nucleic acids, may be employed in the methods and compositions described here. Such libraries may be exposed to the tyrosine kinase, with or without HS1, or HS1, with or without a tyrosine kinase, as described above, and the relevant assay carried out. Depending on the assay, they may also be exposed a cell or organism in the presence of the tyrosine kinase, with or without HS1, or HS 1, with or without a tyrosine kinase.

Selection protocols for isolating desired members of large libraries are known in the art, as typified by phage display techniques. Such systems, in which diverse peptide sequences are displayed on the surface of filamentous bacteriophage (Scott and Smith (1990 *supra*)*,* have proven useful for creating libraries of antibody fragments (and the nucleotide sequences that encoding them) for the *in vitro* selection and amplification of specific antibody fragments that bind a target antigen. The nucleotide sequences encoding the V_{H} and V_{L} regions are linked to gene fragments which encode leader signals that direct them to the periplasmic space of *E. coli* and as a result the resultant antibody fragments are displayed on the surface of the bacteriophage, typically as fusions to bacteriophage coat proteins (e.g., pIII or pVIII). Alternatively, antibody fragments are displayed externally on lambda phage capsids (phagebodies). An advantage of phage-based display systems is that, because they are biological systems, selected library members can be amplified simply by growing the phage containing the selected library member in bacterial cells. Furthermore, since the nucleotide sequence that encodes the polypeptide library member is contained on a phage or phagemid vector, sequencing, expression and subsequent genetic manipulation is relatively straightforward.

Methods for the construction of bacteriophage antibody display libraries and lambda phage expression libraries are well known in the art (McCafferty *et al.* (1990) **supra**; Kang et al. (1991) Proc. Natl. Acad. Sci. U.S.A., 88: 4363; Clackson et al. (1991) Nature, 352: 624; Lowman et al. (1991) Biochemistry, 30: 10832; Burton et al. (1991) Proc. Natl. Acad. Sci U.S.A., 88: 10134; Hoogenboom et al. (1991) Nucleic Acids Res., 19: 4133; Chang et al. (1991) J. Immunol., 147: 3610; Breitling et al. (1991) Gene, 104: 147; Marks *et al.* (1991) supra; Barbas *et al.* (1992) supra; Hawkins and Winter (1992) J. Immunol., 22: 867; Marks et al., 1992, J. Biol. Chem., 267: 16007; Lerner et al. (1992) Science, 258: 1313, incorporated herein by reference). Such techniques may be modified if necessary for the expression generally of polypeptide libraries.

One particularly advantageous approach has been the use of scFv phage-libraries (Bird, R.E., et al. (1988) Science 242: 423-6, Huston et al., 1988, Proc. Natl. Acad. Sci U.S.A., 85: 5879-5883; Chaudhary et al. (1990) Proc. Natl. Acad. Sci U.S.A., 87: 1066-1070; McCafferty *et al.* (1990) supra; Clackson *et al.* (1991) supra; Marks *et al.* (1991) supra; Chiswell *et al.* (1992) Trends Biotech., 10: 80; Marks *et al.* (1992) supra). Various embodiments of scFv libraries displayed on bacteriophage coat proteins have been described. Refinements of phage display approaches are also known, for example as described in WO96/0621 and WO92/01047 (Medical Research Council *et al.*) and WO97/08320 (Morphosys, supra), which are incorporated herein by reference.

Alternative library selection technologies include bacteriophage lambda expression systems, which may be screened directly as bacteriophage plaques or as colonies of lysogens, both as previously described (Huse et al. (1989) Science, 246: 1275; Caton and Koprowski (1990) Proc. Natl. Acad. Sci. U.S.A., 87; Mullinax et al. (1990) Proc. Natl. Acad. Sci. U.S.A., 87: 8095; Persson et al. (1991) Proc. Natl. Acad. Sci. U.S.A., 88: 2432) and are of use. These expression systems may be used to screen a large number of different members of a library, in the order of about 10⁶ or even more. Other screening systems rely, for example, on direct chemical synthesis of library members. One early method involves the synthesis of peptides on a set of pins or rods, such as described in WO84/03564. A similar method involving peptide synthesis on beads, which forms a peptide library in which each bead is an individual library member, is described in U.S. Patent No. 4,631,211 and a related method is described in WO92/00091. A significant improvement of the bead-based methods involves tagging each bead with a unique identifier tag, such as an oligonucleotide, so as to facilitate identification of the amino acid sequence of each library member. These improved bead-based methods are described in WO93/06121.

Another chemical synthesis method involves the synthesis of arrays of peptides (or peptidomimetics) on a surface in a manner that places each distinct library member (e.g., unique peptide sequence) at a discrete, predefined location in the array. The identity of each library member is determined by its spatial location in the array. The locations in the array where binding interactions between a predetermined molecule (e.g., a receptor) and reactive library members occur is determined, thereby identifying the sequences of the reactive library members on the basis of spatial location. These methods are described in U.S. Patent No. 5,143,854; WO90/15070 and WO92/10092; Fodor et al. (1991) Science, 251: 767; Dower and Fodor (1991) Ann. Rep. Med. Chem., 26: 271.

Other systems for generating libraries of polypeptides or nucleotides involve the use of cell-free enzymatic machinery for the *in vitro* synthesis of the library members. In one method, RNA molecules are selected by alternate rounds of selection against a target ligand and PCR amplification (Tuerk and Gold (1990) Science, 249: 505; Ellington and Szostak (1990) Nature, 346: 818). A similar technique may be used to identify DNA sequences which bind a predetermined human transcription factor (Thiesen and Bach (1990) Nucleic Acids Res., 18: 3203; Beaudry and Joyce (1992) Science, 257: 635; WO92/0525 and WO92/14843). In a similar way, *in vitro* translation can be used to synthesise polypeptides as a method for generating large libraries. These methods which generally comprise stabilised polysome complexes, are described further in WO88/08453, WO90/05785, WO90/07003, WO91/02076, WO91/05058, and WO92/02536. Alternative display systems which are not phage-based, such as those disclosed in WO95/22625 and WO95/11922 (Affymax) use the polysomes to display polypeptides for selection. These and all the foregoing documents also are incorporated herein by reference.

### COMBINATORIAL LIBRARIES

Libraries, in particular, libraries of candidate molecules, may suitably be in the form of combinatorial libraries (also known as combinatorial chemical libraries).

A "combinatorial library", as the term is used in this document, is a collection of multiple species of chemical compounds that consist of randomly selected subunits. Combinatorial libraries may be screened for molecules which are capable of acting as an antagonist.

Various combinatorial libraries of chemical compounds are currently available, including libraries active against proteolytic and non-proteolytic enzymes, libraries of whole-cell oncology and anti-infective targets, among others. A comprehensive review of combinatorial libraries, in particular their construction and uses is provided in Dolle and Nelson (1999), Journal of Combinatorial Chemistry, Vol 1 No 4, 235-282. Reference is also made to Combinatorial peptide library protocols (edited by Shmuel Cabilly, Totowa, N.J. : Humana Press, c1998. Methods in Molecular Biology ; v. 87).

Further references describing chemical combinatorial libraries, their production and use include those available from the URL http://www.netsci.org/Science/Combichem/, including The Chemical Generation of Molecular Diversity. Michael R. Pavia, Sphinx Pharmaceuticals, A Division of Eli Lilly (Published July, 1995); Combinatorial Chemistry: A Strategy for the Future - MDL Information Systems discusses the role its Project Library plays in managing diversity libraries (Published July, 1995); Solid Support Combinatorial Chemistry in Lead Discovery and SAR Optimization, Adnan M. M. Mjalli and Barry E. Toyonaga, Ontogen Corporation (Published July, 1995); Non-Peptidic Bradykinin Receptor Antagonists From a Structurally Directed Non-Peptide Library. Sarvajit Chakravarty, Babu J. Mavunkel, Robin Andy, Donald J. Kyle*, Scios Nova Inc. (Published July, 1995); Combinatorial Chemistry Library Design using Pharmacophore Diversity Keith Davies and Clive Briant, Chemical Design Ltd. (Published July, 1995); A Database System for Combinatorial Synthesis Experiments - Craig James and David Weininger, Daylight Chemical Information Systems, Inc. (Published July, 1995); An Information Management Architecture for Combinatorial Chemistry, Keith Davies and Catherine White, Chemical Design Ltd. (Published July, 1995); Novel Software Tools for Addressing Chemical Diversity, R. S. Pearlman, Laboratory for Molecular Graphics and Theoretical Modeling, College of Pharmacy, University of Texas (Published June/July, 1996); Opportunities for Computational Chemists Afforded by the New Strategies in Drug Discovery: An Opinion, Yvonne Connolly Martin, Computer Assisted Molecular Design Project, Abbott Laboratories (Published June/July, 1996); Combinatorial Chemistry and Molecular Diversity Course at the University of Louisville: A Description, Arno F. Spatola, Department of Chemistry, University of Louisville (Published June/July, 1996); Chemically Generated Screening Libraries: Present and Future. Michael R. Pavia, Sphinx Pharmaceuticals, A Division of Eli Lilly (Published June/July, 1996); Chemical Strategies For Introducing Carbohydrate Molecular Diversity Into The Drug Discovery Process.. Michael J. Sofia, Transcell Technologies Inc. (Published June/July, 1996); Data Management for Combinatorial Chemistry. Maryjo Zaborowski, Chiron Corporation and Sheila H. DeWitt, Parke-Davis Pharmaceutical Research, Division of Warner-Lambert Company (Published November, 1995); and The Impact of High Throughput Organic Synthesis on R&D in Bio-Based Industries, John P. Devlin (Published March, 1996).

Techniques in combinatorial chemistry are gaining wide acceptance among modem methods for the generation of new pharmaceutical leads (Gallop, M. A. et al., 1994, J. Med. Chem. 37:1233-1251; Gordon, E. M. et al., 1994, J. Med. Chem. 37:1385-1401.). One combinatorial approach in use is based on a strategy involving the synthesis of libraries containing a different structure on each particle of the solid phase support, interaction of the library with a soluble receptor, identification of the 'bead' which interacts with the macromolecular target, and determination of the structure carried by the identified 'bead' (Lam, K. S. et al., 1991, Nature 354:82-84). An alternative to this approach is the sequential release of defined aliquots of the compounds from the solid support, with subsequent determination of activity in solution, identification of the particle from which the active compound was released, and elucidation of its structure by direct sequencing (Salmon, S. E. et al., 1993, Proc.Natl.Acad.Sci.USA 90:11708-11712), or by reading its code (Kerr, J. M. et al., 1993, J.Am.Chem.Soc. 115:2529-2531; Nikolaiev, V. et al., 1993, Pept. Res. 6:161-170; Ohlmeyer, M. H. J. et al., 1993, Proc.Natl.Acad.Sci.USA 90:10922-10926).

Soluble random combinatorial libraries may be synthesized using a simple principle for the generation of equimolar mixtures of peptides which was first described by Furka (Furka, A. et al., 1988, Xth International Symposium on Medicinal Chemistry, Budapest 1988; Furka, A. et al., 1988,14th International Congress of Biochemistry, Prague 1988; Furka, A. et al., 1991, Int. J. Peptide Protein Res. 37:487-493). The construction of soluble libraries for iterative screening has also been described (Houghten, R. A. et al.1991, Nature 354:84-86). K. S. Lam disclosed the novel and unexpectedly powerful technique of using insoluble random combinatorial libraries. Lam synthesized random combinatorial libraries on solid phase supports, so that each support had a test compound of uniform molecular structure, and screened the libraries without prior removal of the test compounds from the support by solid phase binding protocols (Lam, K. S. et al., 1991, Nature 354:82-84).

Thus, a library of candidate molecules may be a synthetic combinatorial library (e.g., a combinatorial chemical library), a cellular extract, a bodily fluid (e.g., urine, blood, tears, sweat, or saliva), or other mixture of synthetic or natural products (e.g., a library of small molecules or a fermentation mixture).

A library of molecules may include, for example, amino acids, oligopeptides, polypeptides, proteins, or fragments of peptides or proteins; nucleic acids (e.g., antisense; DNA; RNA; or peptide nucleic acids, PNA); aptamers; or carbohydrates or polysaccharides. Each member of the library can be singular or can be a part of a mixture (e.g., a compressed library). The library may contain purified compounds or can be "dirty" (i.e., containing a significant quantity of impurities). Commercially available libraries (e.g., from Affymetrix, ArQule, Neose Technologies, Sarco, Ciddco, Oxford Asymmetry, Maybridge, Aldrich, Panlabs, Pharmacopoeia, Sigma, or Tripose) may also be used with the methods described here.

In addition to libraries as described above, special libraries called diversity files can be used to assess the specificity, reliability, or reproducibility of the new methods. Diversity files contain a large number of compounds (e.g., 1000 or more small molecules) representative of many classes of compounds that could potentially result in nonspecific detection in an assay. Diversity files are commercially available or can also be assembled from individual compounds commercially available from the vendors listed above.

### CANDIDATE SUBSTANCES

Suitable candidate substances include peptides, especially of from about 5 to 30 or 10 to 25 amino acids in size, based on the polypeptides described in the Examples, or variants of such peptides in which one or more residues have been substituted. Peptides from panels of peptides comprising random sequences or sequences which have been varied consistently to provide a maximally diverse panel of peptides may be used.

Furthermore, combinatorial libraries, peptide and peptide mimetics, defined chemical entities, oligonucleotides, and natural product libraries may be screened for activity as inhibitors of binding of a polypeptide to the cell division cycle machinery, for example mitotic/meiotic apparatus (such as microtubules). The candidate substances may be used in an initial screen in batches of, for example 10 substances per reaction, and the substances of those batches which show inhibition tested individually. Candidate substances which show activity in *in vitro* screens such as those described below can then be tested in whole cell systems, such as mammalian cells which will be exposed to the inhibitor and tested for inhibition of any of the stages of the cell cycle.

### ANTIBODIES FOR USE IN METHODS OF PROGNOSIS OR DIAGNOSIS

An antibody useful in the methods of the present invention may comprise a monoclonal antibody or a polyclonal antibody. This section discusses processes for the production of monoclonal or polyclonal antibodies which may be used as antagonists of phosphorylation.

If polyclonal antibodies are desired, a selected mammal (e.g., mouse, rabbit, goat, horse, etc.) is immunised with an immunogenic polypeptide bearing an epitope(s) from a polypeptide, for example, a peptide derived from a tyrosine kinase or HS1 as described above. In particular, immunisation with a heterogenous region of a tyrosine kinase, for example, an N terminal region, or a HS1 binding region of a relevant tyrosine kinase.

Serum from the immunised animal is collected and treated according to known procedures. If serum containing polyclonal antibodies to an epitope from a polypeptide contains antibodies to other antigens, the polyclonal antibodies can be purified by immunoaffinity chromatography. Techniques for producing and processing polyclonal antisera are known in the art. In order that such antibodies may be made, we also provide polypeptides or fragments thereof haptenised to another polypeptide for use as immunogens in animals or humans.

Monoclonal antibodies directed against epitopes in the polypeptides can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against epitopes in the polypeptides can be screened for various properties; i.e., for isotype and epitope affinity.

An alternative technique involves screening phage display libraries where, for example the phage express scFv fragments on the surface of their coat with a large variety of complementarity determining regions (CDRs). This technique is well known in the art.

Antibodies, both monoclonal and polyclonal, which are directed against epitopes from polypeptides are particularly useful in diagnosis, and those which are neutralising are useful in passive immunotherapy. Monoclonal antibodies, in particular, may be used to raise anti-idiotype antibodies. Anti-idiotype antibodies are immunoglobulins which carry an "internal image" of the antigen of the agent against which protection is desired.

Techniques for raising anti-idiotype antibodies are known in the art. These anti-idiotype antibodies may also be useful in therapy.

For the purposes of this document, the term "antibody", unless specified to the contrary, includes fragments of whole antibodies which retain their binding activity for a target antigen. Such fragments include Fv, F(ab') and F(ab')₂ fragments, as well as single chain antibodies (scFv). Furthermore, the antibodies and fragments thereof may be humanised antibodies, for example as described in EP-A-239400.

Antibodies may be used in method of detecting polypeptides present in biological samples by a method which comprises: (a) providing an antibody; (b) incubating a biological sample with said antibody under conditions which allow for the formation of an antibody-antigen complex; and (c) determining whether antibody-antigen complex comprising said antibody is formed. By this process, phosphorylated HS1 may be detected in tissues or samples.

Suitable samples include blood samples.

The antibody antagonist may be bound to a solid support and/or packaged into kits in a suitable container along with suitable reagents, controls, instructions and the like.

### FRAGMENTS, HOMOLOGUES, VARIANT AND DERIVATIVES

It will be appreciated that fragments, homologues, variants and derivatives of the tyorinse kinases or HS1 may themselves have phosphorylation antagonist activity. We therefore disclose the use of such fragments, homologues, variants and derivatives in treatment and/or prevention of disease.

### Homologues

In the context of this document, a "homologous" sequence is taken to include an amino acid sequence which is at least 15, 20, 25, 30, 40, 50, 60, 70, 80 or 90% identical, preferably at least 95 or 98% identical at the amino acid level over at least 10, 15, 20, 25, 30, 40, 50, 60, 70, 80, 90, 100, 110 or 115 amino acids with the target sequence, for example, a peptide isoform specific antagonist of PAK kinase. In particular, homology should typically be considered with respect to those regions of the sequence known to be essential for protein function rather than non-essential neighbouring sequences. This is especially important when considering homologous sequences from distantly related organisms.

Although homology can also be considered in terms of similarity (i.e. amino acid residues having similar chemical properties/functions), in the context of the present document it is preferred to express homology in terms of sequence identity.

Homology comparisons can be conducted by eye, or more usually, with the aid of readily available sequence comparison programs. These publicly and commercially available computer programs can calculate % homology between two or more sequences.

% homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A; Devereux et al., 1984, Nucleic Acids Research 12:387). Examples of other software than can perform sequence comparisons include, but are not limited to, the BLAST package (see Ausubel *et al.,* 1999 *ibid -* Chapter 18), FASTA (Atschul et al., 1990, J. Mol. Biol., 403-410) and the GENEWORKS suite of comparison tools. Both BLAST and FASTA are available for offline and online searching (see Ausubel *et al.,* 1999 *ibid,* pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Advantageously, the BLAST algorithm is employed, with parameters set to default values. The BLAST algorithm is described in detail at
http://www.ncbi.nih.gov/BLAST/blast_help.html, which is incorporated herein by reference. The search parameters are defined as follows, can be advantageously set to the defined default parameters.

Advantageously, "substantial identity" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul (Karlin and Altschul 1990, Proc. Natl. Acad. Sci. USA 87:2264-68; Karlin and Altschul, 1993, Proc. Natl. Acad Sci. USA 90:5873-7; see http://www.ncbi.nih.govBLAST/blast_help.html) with a few enhancements. The BLAST programs are tailored for sequence similarity searching, for example to identify homologues to a query sequence. For a discussion of basic issues in similarity searching of sequence databases, see Altschul et al (1994) Nature Genetics 6:119-129.

The five BLAST programs available at http://www.ncbi.nlm.nih.gov perform the following tasks: **blastp -** compares an amino acid query sequence against a protein sequence database; **blastn -** compares a nucleotide query sequence against a nucleotide sequence database; **blastx -** compares the six-frame conceptual translation products of a nucleotide query sequence (both strands) against a protein sequence database; **tblastn -** compares a protein query sequence against a nucleotide sequence database dynamically translated in all six reading frames (both strands); **tblastx -** compares the six-frame translations of a nucleotide query sequence against the six-frame translations of a nucleotide sequence database.

BLAST uses the following search parameters:
HISTOGRAM - Display a histogram of scores for each search; default is yes. (See parameter H in the BLAST Manual).
DESCRIPTIONS - Restricts the number of short descriptions of matching sequences reported to the number specified; default limit is 100 descriptions. (See parameter V in the manual page).
EXPECT - The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).
CUTOFF - Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.
ALIGNMENTS - Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).
MATRIX - Specify an alternate scoring matrix for BLASTP, BLASTX, TBLASTN and TBLASTX. The default matrix is BLOSUM62 (Henikoff & Henikoff, 1992). The valid alternative choices include: PAM40, PAM120, PAM250 and IDENTITY. No alternate scoring matrices are available for BLASTN; specifying the MATRIX directive in BLASTN requests returns an error response.
STRAND - Restrict a TBLASTN search to just the top or bottom strand of the database sequences; or restrict a BLASTN, BLASTX or TBLASTX search to just reading frames on the top or bottom strand of the query sequence.
FILTER - Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States (1993) Computers and Chemistry 17:191-201, or, for BLASTN, by the DUST program of Tatusov and Lipman (see http://www.ncbi.nlm.nih.gov). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "NNNNNNNNNNNNN") and the letter "X" in protein sequences (e.g., "XXXXXXXXX").

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi - Causes NCBI gi identifiers to be shown in the output, in addition to the accession and/or locus name.

Most preferably, sequence comparisons are conducted using the simple BLAST search algorithm provided at http://www.ncbi.nlm.nih.govBLAST. In some embodiments, no gap penalties are used when determining sequence identity.

Once the software has produced an optimal alignment, it is possible to calculate % homology, preferably % sequence identity. The software typically does this as part of the sequence comparison and generates a numerical result.

### Variants and Derivatives

The terms "variant" or "derivative" in relation to the amino acid sequences disclosed here includes any substitution of, variation of, modification of, replacement of, deletion of or addition of one (or more) amino acids from or to the sequence providing the resultant amino acid sequence retains substantially the same activity as the unmodified sequence. Preferably, the modified sequence has at least one biological activity as the unmodified sequence, preferably all the biological activities of the unmodified sequence. Preferably, the "variant" or "derivative" has the ability to prevent at least some phosphorylation of HS1.

Polypeptides as described in the description and Examples, or fragments or homologues thereof may be modified for use in the methods and compositions described here. Typically, modifications are made that maintain the biological activity of the sequence. Amino acid substitutions may be made, for example from 1, 2 or 3 to 10, 20 or 30 substitutions provided that the modified sequence retains the biological activity of the unmodified sequence. Amino acid substitutions may include the use of non-naturally occurring analogues, for example to increase blood plasma half-life of a therapeutically administered polypeptide.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Polypeptides also include fragments of the peptide antagonists disclosed here. Preferably fragments comprise at least one epitope. Methods of identifying epitopes are well known in the art. Fragments will typically comprise at least 6 amino acids, more preferably at least 10, 20 or more amino acids.

Antagonists, fragments, homologues, variants and derivatives, are typically made by recombinant means. However they may also be made by synthetic means using techniques well known to skilled persons such as solid phase synthesis. The proteins may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the function of the protein of interest sequence. Proteins may also be obtained by purification of cell extracts from animal cells.

The antagonists, variants, homologues, fragments and derivatives disclosed here may be in a substantially isolated form. It will be understood that such polypeptides may be mixed with carriers or diluents which will not interfere with the intended purpose of the protein and still be regarded as substantially isolated. They may also be in a substantially purified form, in which case it will generally comprise the protein in a preparation in which more than 90%, e.g. 95%, 98% or 99% of the protein in the preparation is a protein.

The peptide antagonists of phoshorylation, variants, homologues, fragments and derivatives disclosed here may be labelled with a revealing label. The revealing label may be any suitable label which allows the polypeptide, etc to be detected. Suitable labels include radioisotopes, e.g. ¹²⁵I, enzymes, antibodies, polynucleotides and linkers such as biotin. Labelled polypeptides may be used in diagnostic or prognostic procedures such as immunoassays to determine the amount of a polypeptide in a sample. Polypeptides or labelled polypeptides may also be used in serological or cell-mediated immune assays for the detection of immune reactivity to said polypeptides in animals and humans using standard protocols.

A peptide antagonist, variant, homologue, fragment or derivative disclosed here, optionally labelled, my also be fixed to a solid phase, for example the surface of an immunoassay well or dipstick. Such labelled and/or immobilised polypeptides may be packaged into kits in a suitable container along with suitable reagents, controls, instructions and the like. Such polypeptides and kits may be used in methods of detection of antibodies to the polypeptides or their allelic or species variants by immunoassay.

Immunoassay methods are well known in the art and will generally comprise: (a) providing a polypeptide comprising an epitope bindable by an antibody against said protein; (b) incubating a biological sample with said polypeptide under conditions which allow for the formation of an antibody-antigen complex; and (c) determining whether antibody-antigen complex comprising said polypeptide is formed.

The antagonist, variants, homologues, fragments and derivatives disclosed here may be used in *in vitro* or *in vivo* cell culture systems to study the role of their corresponding genes and homologues thereof in cell function, including their function in disease. For example, truncated or modified polypeptides may be introduced into a cell to disrupt the normal functions which occur in the cell. The polypeptides may be introduced into the cell by *in situ* expression of the polypeptide from a recombinant expression vector (see below). The expression vector optionally carries an inducible promoter to control the expression of the polypeptide.

The use of appropriate host cells, such as insect cells or mammalian cells, is expected to provide for such post-translational modifications (e.g. myristolation, glycosylation, truncation, lapidation and tyrosine, serine or threonine phosphorylation) as may be needed to confer optimal biological activity on recombinant expression products. Such cell culture systems in which the peptide antagonist, variants, homologues, fragments and derivatives disclosed here are expressed may be used in assay systems to identify candidate substances which interfere with or enhance the functions of the polypeptides in the cell.

### PHARMACEUTICAL COMPOSITIONS

While it is possible for the composition comprising the antagonist of phosphorylation or dephosphorylation agent, for example, a peptide, or nucleic acid encoding these, or a small molecule, to be administered alone, it is preferable to formulate the active ingredient as a pharmaceutical formulation. We therefore also disclose pharmaceutical compositions comprising antagonists of phosphorylation and agonists of dephosphorylation. Such pharmaceutical compositions are useful for delivery of such antagonists and agonists to an individual for the treatment or alleviation of symptoms as described.

The composition may include the antagonist or agonist, a structurally related compound, or an acidic salt thereof. The pharmaceutical formulations comprise an effective amount of antagonistor angonist, together with one or more pharmaceutically-acceptable carriers. An "effective amount" of an antagonist or agnoist is the amount sufficient to alleviate at least one symptom of a disease as described above.

The effective amount will vary depending upon the particular disease or syndrome to be treated or alleviated, as well as other factors including the age and weight of the patient, how advanced the disease etc state is, the general health of the patient, the severity of the symptoms, and whether the antagonist or agonist is being administered alone or in combination with other therapies.

Suitable pharmaceutically acceptable carriers are well known in the art and vary with the desired form and mode of administration of the pharmaceutical formulation. For example, they can include diluents or excipients such as fillers, binders, wetting agents, disintegrators, surface-active agents, lubricants and the like. Typically, the carrier is a solid, a liquid or a vaporizable carrier, or a combination thereof. Each carrier should be "acceptable" in the sense of being compatible with the other ingredients in the formulation and not injurious to the patient. The carrier should be biologically acceptable without eliciting an adverse reaction (e.g. immune response) when administered to the host.

The invention is described further, for the purpose of illustration only, in the following examples.

### Example 1- Tissue samples and cell purification

Leukemic lymphocytes were obtained from the peripheral blood of 40 CLL patients, diagnosed according to the National Cancer Institute-Working Group (NCI-WG)(38). The following parameters were analysed for each patient: CD38 expression(39), the IgV_{H} mutational status(39), Zap-70 expression(22), disease stage according to Binet(40) or Rai modified criteria(41), history of treatment, progressive or stable disease as defined by the NCI-WG(42) and survival. All patients were either untreated or off-therapy since at least six months.

Leukemic CD19⁺ cells were purified using a B-lymphocyte enrichment kit (RosetteSep^{™}, StemCell Technologies), following manufacturer's instructions. Purity of all preparations was always above 99%, and the cells were co-expressing CD 19 and CD5 on their cell surface as checked by flow-cytometry (FC500 - Beckman Coulter), and the preparations were virtually devoid of NK and T lymphocytes. The purified cells, at the concentration of 25x10⁶ (1mg protein/sample, as checked by BCA assay) were washed with PBS, pelleted and stored at -20°C.

Tonsillar B cells from discarded tissue were stained with anti IgDFITC (Southern Biotechnology Associates), anti-CD19PE and anti-CD38PC5 (Beckman-Coulter) and then FACS-purified on a Coulter Altra (Beckman-Coulter) in order to obtain Naive B cells (CD19⁺, IgD⁺, CD38⁻) and Memory B cells (CD19⁺, IGD⁻, CD38⁻)(43). Purity was >98%.

All tissue samples were obtained following institutional guidelines at our institutions.

### Example 2 - Cell culture and stimulation

Purified normal and leukemic B cells were cultured in RPMI 1640 medium supplemented with 10% FCS, 2mM L-glutamine and 15 µg/ml gentamicin (complete RPMI - cRPMI) (Invitrogen-Lifetechnologies), at the concentration of 3x10⁶ cells/ml, in the presence or the absence of goat F(ab¹)2 anti-human IgM antibodies (Caltag laboratories) (20µg/ml), for 2 or 5 minutes. Cells were then lysed, pelletted and stored at -80°C.

### Example 3 - Mono and Two-Dimensional Electrophoresis (2-DE)

The pellet lyophilised and solubilised with 2-DE buffer [9M Urea, 10mM Tris, 4% CHAPS, 65mM DTT, 2% IPG buffer ampholine pH 4-7 (Amersham Biosciences), protease inhibitor cocktail]. Protein samples, 1mg or 250mg, were applied to 18 or 7cm IPGstrips pH 4-7 (Amersham Biosciences) respectively, by in-gel rehydration. Iso-electro Focusing (IEF) was performed with an IPGphor system (Amersham Biosciences) following standard protocol as described in Conti et al(44). Strips were equilibrated in 50 mM pH 8.8 Tris-HCl buffer containing 6 M urea, 30% glycerol, 2% SDS and 2% DTT, followed by an incubation in the same buffer replacing DTT with 2.5% iodoacetamide. The strips were loaded on the top of either 9-16% gradient or 10% acrylamide SDS-PAGE gels for the second dimension separation.

Protein spots were visualized by staining gels with MS compatible silver stain(45). Imagines were acquired at high resolution using a Molecular Dynamics Personal SI Laser Densitometer (Amersham Biosciences) and 2-D protein patterns analyzed using ImageMaster 2D Platinum 5.0 software (Amersham Biosciences) or Progenesis Workstation, V. 2004 (Nonlinear Dynamics). Relative molecular masses (Mr) were estimated by comparison with Mr reference markers (Precision, Bio-Rad) and pI values assigned to detected spots by calibration as described in the Amersham Biosciences guide lines.

Monodimensional SDS-PAGE were performed solubilizing the cell pellets with Laemmli buffer and loading 10 µg of protein into 10 % acrylamide gel.

### Example 4 - Immunoprecipitation and Western blot analysis

Total cell lysates were incubated overnight at 4°C with anti-phosphotyrosine antibody 4G10 (Upstate Biotechnology) immobilized on protein G-coated Sepharose Beads (Amersham Biosciences). After a quick spin the left over was collected and the beads were washed 3 times with lysis buffer. Immunocomplexes were recovered by incubation with Laemmli buffer (5' at 95°C).

Immuno-precipitated or whole proteins, from cells incubated with or without anti-IgM stimulation, were resolved either by SDS-PAGE or 2-DE, electron-transferred onto nitrocellulose membranes and incubated 2 hrs with one of the following antibodies (1:500 to 1:1000 working dilution): monoclonal anti-HS1, (BD-Transduction laboratories), monoclonal anti-Syk, rabbit polyclonal anti-Lyn (Upstate Biotechnology and anti-β actin (Sigma-Aldrich Srl).

Immunoreactivity was revealed by incubation with either goat anti-rabbit Ig or rabbit antimouse Ig conjugated with HRP (Upstate Biotechnology), followed by ECL (Amersham Biosciences) reaction and film exposures.

### Example 5 - Protein Identification by MALDI-TOF MS analysis

Spots of interest were excised from gels (either by manual or automated excision (ProXCISION; PerkinElmer); reduced, alkylated and digested overnight with bovine trypsin (Roche), as previously described(46). One µl aliquots of the supernatant were used for mass spectrometry analysis using the dried droplet technique and α-cyano-4-hydroxycinnamic acid as matrix. Mass spectra were obtained on a MALDI-TOF Voyager-DE STR (Applied Biosystem) mass spectrometer. Alternatively, gel fragments were further extracted and resulting peptides mixture subjected to a single desalting/concentration step before mass spectrometric analysis over µZipTipC18 (Millipore Corporation). Spectra were internally calibrated using trypsin autolysis products and processed via Data Explorer software. Proteins were unambiguously identified by searching a comprehensive non-redundant protein database using the programs ProFound(47) and Mascot(47). One missed cleavage per peptide was allowed, and an initial mass tolerance of 50 ppm was used in all searches.

### Example 6 - Phosphatase treatment

λ-PPase (NewEngland BioLabs) treatment was performed as described in Yamagata et al(28) with modifications. In brief, pelleted cells (25x10⁶) were resuspended 12 hrs in lysis buffer (1% w/v NP-40, 1% w/v SDS, 50 mM Tris pH 7.6, 150 mM NaCl protease inhibitor cocktail). Sixty µl of lysate, corresponding to 600µg of protein, were bring to a final volume of 600 µl with deionized water followed by addition of 20 µl of 20mM MnCl₂ solution and 20 µl of λ-PPase buffer. For each addition, the solution was gently mixed. The mixture was divided into two aliquots, and 300 units of λ-PPase was added to one of the aliquots. After mixing, aliquots were incubated for 7 hrs or 15 hrs at 30°C. Proteins were acetone precipitated at -20°C and used for 2-DE analysis.

### Example 7 - Statistical analysis

Fisher exact test (2-tailed) was performed to determine the degree of association among patients' subsets (with mutated/unmutated IgV_{H}, negative/positive for CD38, negative/positive for ZAP70, with one/two HS1 spots). Cumulative survival analysis were obtained according to Kaplan-Meier method.

### Example 8 - 2-D electrophoresis identifies distinct patterns of protein expression in CLL patient subsets

To minimize inter-patient variations we started by investigating a limited number of highly selected CLL patients (14 cases) who were completely concordant for IgV_{H} mutational status (unmutated vs mutated), CD38 expression (positive vs negative) and clinical behavior (progressive vs stable disease) (Table 1, upper panel). Seven patients carried unmutated IgV_{H} genes, were CD38⁺ and experienced clinical progression requiring treatment during their clinical course (bad prognosis subset). The other 7 patients had mutated IgV_{H} genes, were negative for CD38 and had a stable disease with a long follow-up (median time 102 months) (good prognosis subset). CD19⁺ CD5⁺ purified leukemic cells were lysed and proteins resolved on 2-DE, and visualized by silver staining. The protein profile analysis on silver-stained gels showed a number of spots differentially expressed in the samples obtained from the two CLL subsets. We focussed our attention on two close spots with the same relative molecular mass (Mr) of 79 kD and different isoelectric point (pI) (4,83 and 4,86 respectively) (Figure 1a) which were both expressed in the 2-D gels obtained from most good prognosis patients (Figure 1b Subset 1; Table 1), while the more acidic protein prevailed in the preparations from the bad prognosis subset of patients (Figure 1b Subset 2; Table 1). Though the number of cases studied was small, 5/7 of progressive but only 1/7 of stable cases had one spot. Differences in protein expression pattern were statistically significant with a p < 0,05 using a Chi-square test.

### Example 9 - Mass spectrometry analysis identifies HS1 as the protein differentially expressed in the two subsets of CLL patients

The two protein spots were excised from 2D-gels and analyzed by Matrix Assisted Laser Desorption Ionization-Time Of Flight (MALDI-TOF) MS, after tryptic digestion. The two spots resulted to be the same protein and were identified as HS1 (Table 2). This suggested that the spots represent either structural or functional isoforms of the same protein. To confirm the identification results, 2-D gels from 5 representative patients were transferred to nitrocellulose and incubated with a monoclonal anti-HS1 antibody that hybridized specifically at the same mass values and isoelectric point of both protein spots (Figure 1c).

Western blot analysis on SDS-PAGE gels was performed with anti-HS1 to define the expression levels of the molecule irrespective of its functional modifications. This analysis showed that the protein was expressed in equal amounts in both CLL subsets as were the signalling molecules Syk and Lyn(11,12)(data not shown).

### Example 10 - Treatment with phosphatase of CLL cell lysates demonstrates the presence of phosphorylation on the differentially expressed HS1 protein spots

The different pI of the two (left and right) HS1 protein spots (Figure 1) suggested the possibility that posttranslational modifications might be responsible for the differential expression of HS1 revealed by the 2-DE.

As it has been shown that HS1 may become phosphorylated at several tyrosine residues following BCR stimulation(24-27) resulting in a more acidic pI, we aimed at defining whether the different HS1 migration pattern in 2-DE might be caused by protein phosphorylation.

We performed dephosphorylation experiments using λ protein phosphatase (λPPase) that removes phosphoryl groups from different residues (Tyr, Ser, Thr, His) (28). CLL cell lysates from purified cells of 4 representative cases (2 from each group) were incubated in the presence of λPPase at different incubation times. After 7 and 15 hours of incubation, 2-DE and Western blot with anti-HS1 antibody (Figure 2) revealed a shift of the more acidic isoforms toward a less acidic pI. This documents that the HS1 left spot is a phosphorylated form of the protein and that the HS1 right spot corresponds to a dephosphorylated molecule (Figure 2). Hence these data indicate that most of the HS1 protein present in the cells from bad prognosis CLL patients is constitutively phosphorylated, whereas only a fraction of HS1 is phosphorylated in the cells from the good prognosis subset.

In particularly well resolved 2-DE it was possible to detect several intermediate HS 1 isoforms likely corresponding to different rates of phosphorylation (Figure 2, Subset 1). These spots following λPPase incubation shifted through successive intermediates from more acidic and heavy isoforms to less acidic and lighter isoform/s (Figure 2, Subset 1).

### Example 11 - HS1 expression pattern correlates with CD38 expression and IgV_{H} mutational status in a larger cohort of CLL patients

We expanded the data obtained in the highly selected group of patients above described by examining the expression of HS1 in 26 further unselected cases who were more heterogeneous in terms of biological prognostic factors and clinical course (Table 1, lower panel). Overall, in the 40 patients studied for HS1 expression (Table 1), the expression of CD38 had a significant correlation with IgV_{H} mutational status (p<0.001) and the expression of Zap-70 correlated with IgV_{H} mutational status (p=0.003). In these patients the differential pattern of HS1 expression (Table 1) was found to correlate with both the expression of CD38 (p=0.022) and IgV_{H} mutations (p=0.008). Rather unexpectedly no significant correlation was found with the expression of Zap-70 (p=0.505). When the 40 patients were grouped according to the HS1 profile it appeared that those presenting 1 single HS1 spot had a median survival time (184 months) significantly shorter than those with two spots, (median survival not reached; p=0.024) (Fig 3).

### Example 12 - Modifications of Syk and HS1 upon stimulation of the BCR in leukemic and normal B cells

CLL cells respond differently to BCR stimulation, depending on their biological featureswith unmutated and/or CD38 and/or ZAP-70 positive cases being better responders than mutated and/or CD38 and/or ZAP-70 negative cases(11, 12, 16, 22, 23). Therefore, we planned to investigate whether an antigen-like BCR stimulation might modify the phosphorylation status of Syk and HS1 molecules involved in the downstream signaling pathway.

First, we studied the phosphorylation status of Syk in 10/14 "fully concordant" CLL cases (Table 1, upper panel), after an in vitro culture of 5' and immunoprecipitation. At the constitutive level, the levels of phosphorylation of Syk protein were similar in all cases irrespective of their biological features. After stimulation with anti-IgM antibodies, 4/5 unmutated/CD38⁺ cases increased the phosphorylated form of the protein, while no mutated/CD38⁻ cases did so (data not shown), confirming previously published results(11, 12,22).

To investigate whether HS1 protein could be modified after a similar antigen-like BCR stimulation, both CLL leukemic and normal mature B lymphocytes purified from tonsils were cultured for 2' in the presence or the absence of anti-IgM and then immediately harvested for protein separation. To allow the evaluation of potential modifications of the phosphorylated forms we selected four CLL cases with two spots, two mutated/CD38⁻ and two unmutated/CD38⁺. In these experimental conditions the capacity of CLL cells to increase the phosphorylation levels of HS1 were variable. As expected (11, 12) the two mutated/CD38⁻ cases did not modify their two spots pattern, while 1/2 unmutated/CD38⁺ cases with two spots showed an increase in the phosphorylated left spot (data not shown). In normal B cells, 2-DE allowed to visualize a shift of the spot representing the non- or less-phosphorylated form of HS1 (right spot) toward a more acidic position (left spot) representing the highly-phosphorylated form (Figure 4a).

We then analyzed the proteomic profile of ex-vivo purified naïve and memory tonsillar B lymphocytes (purity >98%) that differ by their in vivo antigenic experience. Naive B cells showed the presence of both HS1 forms (left and right spots) at equal levels while memory B cells expressed mainly the phosphorylated fraction (left spot) (Figure 4b). This confirms that also in vivo antigen activation may be responsible for the differential aspect of HS1 migration pattern in 2-D gels. Western blot analysis of protein lysates obtained from both Naive and memory B cells showed similar levels of total HS1 protein (data not shown), regardless of their functional modification.

### Example 13 - Production of a monoclonal antibody against the phosphorylated form of HS1

We followed the standard procedure for the production of monoclonal antibodies which included the following phases:

### 1) Antigen synthesis

Two peptides were synthesized with and without phosphorylation on the relevant tyrosine residues (Y378 and Y397) (purity>70%) with the following sequence:
NH2-C-PENDYEDVEEMDR-COOH
MH2-C-PEGDYEEVLE

The phosphorylated peptides were conjugated with two different carrier proteins (KLH to immunize and OVA to perform the sceening).The two non-phosphorylated peptides were coniugated only with OVA.

### 2) Antibody production

Balb/c mice (3mice/antigen) were immunized with a mixture of the two phosphorylated peptides coniugated with KLH. An ELISA test was set-up to follow the immunization. Animals sera were checked for positivity (cut-off value> 1.10000) and somatic cell hybrid fusion was performed. A first screening of the hybridomas was performed by ELISA and the positive ones were selected (9 clones). Positivity was confirmed using a differential ELISA analysis, using both phosphorylated and non-phosphorylated peptides. Positive clones were purified and frozen in liquid nitrogen.

### Results and discussion:

We immunized mice with a mixture of two peptides conjugated with a carrier and we performed a somatic cell hybrid fusion. Nine positives clones against HS1 were obtained. A differential screening made by ELISA (Fig.7) using phosphorylated and non-phosphorylated peptides showed that 7 clones were specific for the phosphorylated form and the remaining two recognized both phosphorylated and non- phosphorylated HS 1.

A successive screening showed that all clones were specific for the peptide containing phosphorylated Y378.

In order to validate the positive clones, the supernatants in WB may be tested and we have now evidence that all 9 supernatants recognize HS1. The supernatants may also be tested by flow-citometry, immunoprecipitation, immunofluorescence and immunohistochemistry analysis.

Various modifications and variations of the described methods and system of the invention will be apparent to those skilled in the art. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the claims.

**Table 1**

| Clinical and biological features of the CLL patients | | | | | | | | | | | | |
|---|---|---|---|---|---|---|---|---|---|---|---|---|
| **Case #** | **Age (years)** | **Sex** | **HC^{A}** | **LC^{H}** | **Clinical course** | **IgV_{H}^{C} (%)** | **CD38 (%)** | **ZAP 70 expression^{D}** | **Stage'** | **Follow-up^{F}** | **Death^{G}** | **HS1 spots^{H}** |
| 1 | 79 | M | IgM/D | kappa | progressive | 100,0 | 41 | Pos | 0/A | 97 | yes | 1 |
| 2 | 69 | M | IgM/D | lambda | progressive | 100,0 | 81 | Pos | ND | 36 | no | 2 |
| 3 | 53 | M | IgM/D | kappa | progressive | 100,0 | 89 | Pos | 2/B | 48 | yes | 1 |
| 4 | 66 | M | IgM/D | kappa | progressive | 100,0 | 99 | Pos | 4/C | 107 | no | 1 |
| 5 | 82 | M | IgM/D | lambda | progressive | 99,0 | 60 | Pos | 2/B | 162 | no | 1 |
| 6 | 80 | F | IgM/D | lambda | progressive | 98,6 | 30 | Pos | 2/B | 84 | yes | 1 |
| 7 | 79 | M | IgM/D | kappa | progressive | 98,6 | 32 | Pos | 2/B | 82 | no | 2 |
| 8 | 53 | M | IgM/D | kappa | stable | 96,2 | 0,1 | Neg | 1/B | 159 | no | 2 |
| 9 | 55 | M | IgM/D | lambda | stable | 95,3 | 0,3 | Neg | 0/A | 107 | no | 1 |
| 10 | 50 | M | IgM/D | kappa | stable | 93,3 | 1,5 | Neg | 0/A | 105 | no | 2 |
| 11 | 71 | F | IgM/D | kappa | stable | 92,9 | 3,0 | Neg | 0/A | 199 | no | 2 |
| 12 | 67 | F | IgM/D | lambda | stable | 91,8 | 0,2 | Neg | 0/A | 143 | no | 2 |
| 13 | 78 | F | IgM/D | kappa | stable | 89,5 | 0,4 | Neg | 0/A | 101 | no | 2 |
| 14 | 72 | F | IgM/D | lambda | stable | 88,4 | 0,1 | Neg | 0/A | 53 | no | 2 |
| 15 | 63 | M | IgM/D | kappa | progressive | 100,0 | 93 | Neg | 0/A | 98 | yes | 1 |
| 16 | 73 | M | IgM/D | kappa | progressive | 100,0 | 78 | Neg | 0/A | 117 | no | 1 |
| 17 | 85 | F | IgM/D | lambda | progressive | 99,3 | 0,5 | Neg | 1/A | 44 | no | 1 |
| 18 | 76 | F | IgM/D | kappa | progressive | 99,0 | 38 | Pos | 0/A | 59 | no | 2 |
| 19 | 76 | M | IgM/D | kappa | progressive | 99,0 | 74 | Pos | 0/A | 101 | no | 2 |
| 20 | 76 | F | IgM/D | kappa | progressive | 98,6 | 100, | Pos | 2/B | 13 | no | 1 |
| 21 | 57 | F | IgM/D | kappa | progressive | 98,3 | 33 | Pos | 2/B | 68 | no | 1 |
| 22 | 83 | F | IgM/D | lambda | progressive | 97,6 | 86 | Neg | 0/A | 97 | no | 2 |
| 23 | 72 | M | IgM/D | kappa | progressive | 96,6 | 16 | Neg | 4/C | 81 | no | 2 |
| 24 | 76 | M | IgM | lambda | progressive | 94,3 | 0,6 | Neg | 0/A | 158 | yes | 2 |
| 25 | 68 | M | IgM/D | lambda | progressive | 93,5 | 0,4 | Pos | 0/A | 163 | no | 2 |
| 26 | 80 | F | IgG | lambda | progressive | 91,2 | 0,4 | Neg | 0/A | 222 | no | 2 |
| 27 | 78 | F | IgM/D | lambda | progressive | 88,5 | 100 | Neg | 0/A | 180 | yes | 1 |
| 28 | 73 | M | IgM/D | lambda | progressive | ND | 53 | Neg | 0/A | 81 | no | 1 |
| 29 | 62 | F | IgM/D | lambda | stable | 99,7 | 3,7 | Pos | 0/A | 44 | no | 1 |
| 30 | 78 | M | IgM/D | kap/lam | stable | 97,0 | 46 | Neg | 0/A | 57 | no | 1 |
| 31 | 69 | F | IgM/D | kappa | stable | 96,7 | 0,7 | Pos | 0/A | 84 | no | 2 |
| 32 | 68 | F | IgM/D | kappa | stable | 94,9 | 0,3 | Neg | 0/A | 91 | no | 1 |
| 33 | 62 | M | IgM/D | lambda | stable | 94,7 | 0,4 | Neg | 0/A | 73 | no | 2 |
| 34 | 70 | M | IgM/D | kappa | stable | 93,7 | 31 | Pos | 0/A | 58 | no | 1 |
| 35 | 62 | M | IgM/D | lambda | stable | 93,2 | 1,1 | Neg | 0/A | 228 | no | 2 |
| 36 | 74 | M | IgM/D | kappa | stable | 92,5 | 1,0 | Pos | 2/A | 125 | no | 2 |
| 37 | 61 | M | IgM/D | lambda | stable | 89,5 | 1,0 | Pos | 0/A | 182 | no | 2 |
| 38 | 61 | F | IgG | kappa | stable | 88,7 | 0,1 | Neg | 1/A | 182 | no | 1 |
| 39 | 74 | M | IgM/D | kappa | stable | 87,7 | 3,0 | Pos | 0/A | 95 | no | 1 |
| 40 | 73 | F | IgM | kappa | stable | ND | 0,8 | Pos | 0/A | 35 | no | 1 |

| | | | | | | | | | | | | |
|---|---|---|---|---|---|---|---|---|---|---|---|---|
| ^{G}Heavy chain, ^{B}Light chain, ^{C}% of somatic mutations in the IgV_{H} genes, ^{D}positive (pos) or negative (neg) as determined by immunoblot analysis, ^{E}according to Rai/Binet, ^{F}months, ^{G}CLL related events, ^{H}number of spots revealed on silver stained 2D gels: 1=single left spot, 2=right+left spot. ND: not determined | | | | | | | | | | | | |

**Table 2**

| **Proteins identified by MALDI-TOF MS analysis** | | | | | | | |
|---|---|---|---|---|---|---|---|
| | | | **pI/Mr** | | | | |
| **spot** | **Protein name** | **Accession number** | **exp.** | **Theor.** | **match. pep.** | **seq. cov.** | **Mascot score** |
| **Right** | Hematopoietic lineage cell specific protein 1(HS1) | gi 123577 | 4.83/79432 | 4.74/53998 | 19 | 43% | 184 |
| **Left** | Hematopoietic lineage cell specific protein 1(HS1) | gi 123577 | 4.86/79212 | 4.74/53998 | 18 | 36% | 172 |

| | | | | | | | |
|---|---|---|---|---|---|---|---|
| MALDI-TOF= Matrix Assisted Laser Desorption Ionization-Time Of Flight; MS= Mass Spectrometry; pI/Mr= Isoelectric point / relative molecular mass; exp.= experimental; theor.= theoretical; match. pep.= matching peptides; seq. cov.= sequence coverage. | | | | | | | |

### References

1. Fais, F., Ghiotto, F., Hashimoto, S., Sellars, B., Valetto, A., Allen, S.L., Schulman, P., Vinciguerra, V.P., Rai, K., Rassenti, L.Z., et al. 1998. Chronic lymphocytic leukemia B cells express restricted sets of mutated and unmutated antigen receptors. J Clin Invest 102:1515-1525.
2. Hamblin, T.J., Davis, Z., Gardiner, A., Oscier, D.G., and Stevenson, F.K. 1999. Unmutated Ig V(H) genes are associated with a more aggressive form of chronic lymphocytic leukemia. Blood 94:1848-1854.
3. Kipps, T.J., Tomhave, E., Pratt, L.F., Duffy, S., Chen, P.P., and Carson, D.A. 1989. Developmentally restricted immunoglobulin heavy chain variable region gene expressed at high frequency in chronic lymphocytic leukemia. Proc Natl Acad Sci U S A 86:5913-5917.
4. Schroeder, H.W., Jr., and Dighiero, G. 1994. The pathogenesis of chronic lymphocytic leukemia: analysis of the antibody repertoire. Immunol Today 15:288-294.
5. Ghia, P., Stamatopoulos, K., Belessi, C., Moreno, C., Stella, S., Guida, G., Michel, A., Crespo, M., Laoutaris, N., Montserrat, E., et al. 2004. Geographical patterns and pathogenetic implications of IGHV gene usage in chronic lymphocytic leukemia: the lesson of the IGHV3-21 gene. Blood 5:5.
6. Tobin, G., Thunberg, U., Karlsson, K., Murray, F., Laurell, A., Willander, K., Enblad, G., Merup, M., Vilpo, J., Juliusson, G., et al. 2004. Subsets with restricted immunoglobulin gene rearrangement features indicate a role for antigen selection in the development of chronic lymphocytic leukemia. Blood 104:2879-2885. Epub 2004 Jun 2824.
7. Widhopf, G.F., 2nd, Rassenti, L.Z., Toy, T.L., Gribben, J.G., Wierda, W.G., and Kipps, T.J. 2004. Chronic lymphocytic leukemia B cells of more than 1% of patients express virtually identical immunoglobulins. Blood 104:2499-2504. Epub 2004 Jun 2424.
8. Messmer, B.T., Albesiano, E., Efremov, D.G., Ghiotto, F., Allen, S.L., Kolitz, J., Foa, R., Damle, R.N., Fais, F., Messmer, D., et al. 2004. Multiple distinct sets of stereotyped antigen receptors indicate a role for antigen in promoting chronic lymphocytic leukemia. J Exp Med 200:519-525.
9. Damle, R.N., Ghiotto, F., Valetto, A., Albesiano, E., Fais, F., Yan, X.J., Sison, C.P., Allen, S.L., Kolitz, J., Schulman, P., et al. 2002. B-cell chronic lymphocytic leukemia cells express a surface membrane phenotype of activated, antigen-experienced B lymphocytes. Blood 99:4087-4093.
10. Damle, R.N., Wasil, T., Fais, F., Ghiotto, F., Valetto, A., Allen, S.L., Buchbinder, A., Budman, D., Dittmar, K., Kolitz, J., et al. 1999. Ig V gene mutation status and CD38 expression as novel prognostic indicators in chronic lymphocytic leukemia. Blood 94:1840-1847.
11. Chen, L., Widhopf, G., Huynh, L., Rassenti, L., Rai, K.R., Weiss, A., and Kipps, T.J. 2002. Expression of ZAP-70 is associated with increased B-cell receptor signaling in chronic lymphocytic leukemia. Blood 100:4609-4614.
12. Lanham, S., Hamblin, T., Oscier, D., Ibbotson, R., Stevenson, F.K., and Packham, G. 2002. Differential signaling via surface IgM is associated with VH gene mutational status and CD38 expression in chronic lymphocytic leukemia. Blood 26:26.
13. Vilen, B.J., Nakamura, T., and Cambier, J.C. 1999. Antigen-stimulated dissociation of BCR mIg from Ig-alpha/Ig-beta: implications for receptor desensitization. Immunity 10:239-248.
14. Deaglio, S., Capobianco, A., Bergui, L., Durig, J., Morabito, F., Duhrsen, U., and Malavasi, F. 2003. CD38 is a signaling molecule in B-cell chronic lymphocytic leukemia cells. Blood 102:2146-2155. Epub 2003 May 2122.
15. Hamblin, T.J., Orchard, J.A., Ibbotson, R.E., Davis, Z., Thomas, P.W., Stevenson, F.K., and Oscier, D.G. 2002. CD38 expression and immunoglobulin variable region mutations are independent prognostic variables in chronic lymphocytic leukemia, but CD38 expression may vary during the course of the disease. Blood 99:1023-1029.
16. Zupo, S., Isnardi, L., Megna, M., Massara, R., Malavasi, F., Dono, M., Cosulich, E., and Ferrarini, M. 1996. CD38 expression distinguishes two groups of B-cell chronic lymphocytic leukemias with different responses to anti-IgM antibodies and propensity to apoptosis. Blood 88:1365-1374.
17. Zupo, S., Massara, R., Dono, M., Rossi, E., Malavasi, F., Cosulich, M.E., and Ferrarini, M. 2000. Apoptosis or plasma cell differentiation of CD38-positive B-chronic lymphocytic leukemia cells induced by cross-linking of surface IgM or IgD. Blood 95:1199-1206.
18. Chen, L., Apgar, J., Huynh, L., Dicker, F., Giago-McGahan, T., Rassenti, L., Weiss, A., and Kipps, T.J. 2004. ZAP-70 directly enhances IgM signaling in chronic lymphocytic leukemia. Blood 28:28.
19. Chan, A.C., Iwashima, M., Turck, C.W., and Weiss, A. 1992. ZAP-70: a 70 kd protein-tyrosine kinase that associates with the TCR zeta chain. Cell 71:649-662.
20. Crespo, M., Bosch, F., Villamor, N., Bellosillo, B., Colomer, D., Rozman, M., Marce, S., Lopez-Guillermo, A., Campo, E., and Montserrat, E. 2003. ZAP-70 expression as a surrogate for immunoglobulin-variable-region mutations in chronic lymphocytic leukemia. N Engl JMed 348:1764-1775*.*
21. Rassenti, L.Z., Huynh, L., Toy, T.L., Chen, L., Keating, M.J., Gribben, J.G., Neuberg, D.S., Flinn, I.W., Rai, K.R., Byrd, J.C., et al. 2004. ZAP-70 compared with immunoglobulin heavy-chain gene mutation status as a predictor of disease progression in chronic lymphocytic leukemia. NEngl J Med 351:893-901.
22. Wiestner, A., Rosenwald, A., Barry, T.S., Wright, G., Davis, R.E., Henrickson, S.E., Zhao, H., Ibbotson, R.E., Orchard, J.A., Davis, Z., et al. 2003. ZAP-70 expression identifies a chronic lymphocytic leukemia subtype with unmutated immunoglobulin genes, inferior clinical outcome, and distinct gene expression profile. Blood 101:4944-4951. Epub 2003 Feb 4920.
23. Rosenwald, A., Alizadeh, A.A., Widhopf, G., Simon, R., Davis, R.E., Yu, X., Yang, L., Pickeral, O.K., Rassenti, L.Z., Powell, J., et al. 2001. Relation of gene expression phenotype to immunoglobulin mutation genotype in B cell chronic lymphocytic leukemia. J Exp Med 194:1639-1647.
24. Yamanashi, Y., Okada, M., Semba, T., Yamori, T., Umemori, H., Tsunasawa, S., Toyoshima, K., Kitamura, D., Watanabe, T., and Yamamoto, T. 1993. Identification of HS1 protein as a major substrate of protein-tyrosine kinase(s) upon B-cell antigen receptor-mediated signaling. Proc Natl Acad Sci USA 90:3631-3635.
25. Yamanashi, Y., Fukuda, T., Nishizumi, H., Inazu, T., Higashi, K., Kitamura, D., Ishida, T., Yamamura, H., Watanabe, T., and Yamamoto, T. 1997. Role of tyrosine phosphorylation of HS1 in B cell antigen receptor-mediated apoptosis. J Exp Med 185:1387-1392.
26. Brunati, A.M., Donella-Deana, A., James, P., Quadroni, M., Contri, A., Marin, O., and Pinna, L.A. 1999. Molecular features underlying the sequential phosphorylation of HS1 protein and its association with c-Fgr protein-tyrosine kinase. J Biol Chem 274:7557-7564.
27. Ruzzene, M., Brunati, A.M., Marin, O., Donella-Deana, A., and Pinna, L.A. 1996. SH2 domains mediate the sequential phosphorylation of HS1 protein by p72syk and Src-related protein tyrosine kinases. Biochemistry 35:5327-5332.
28. Yamagata, A., Kristensen, D.B., Takeda, Y., Miyamoto, Y., Okada, K., Inamatsu, M., and Yoshizato, K. 2002. Mapping of phosphorylated proteins on two-dimensional polyacrylamide gels using protein phosphatase. Proteomics 2:1267-1276.
29. Klein, U., Tu, Y., Stolovitzky, G.A., Mattioli, M., Cattoretti, G., Husson, H., Freedman, A., Inghirami, G., Cro, L., Baldini, L., et al. 2001. Gene expression profiling of B cell chronic lymphocytic leukemia reveals a homogeneous phenotype related to memory B cells. J Exp Med 194:1625-1638.
30. Hata, D., Nakamura, T., Kawakami, T., Kawakami, Y., Herren, B., and Mayumi, M. 1994. Tyrosine phosphorylation of MB-1, B29, and HS1 proteins in human B cells following receptor crosslinking. Immunol Lett 40:65-71.
31. Taniuchi, I., Kitamura, D., Maekawa, Y., Fukuda, T., Kishi, H., and Watanabe, T. 1995. Antigen-receptor induced clonal expansion and deletion of lymphocytes are impaired in mice lacking HS1 protein, a substrate of the antigen-receptor-coupled tyrosine kinases. Embo J 14:3664-3678.
32. Hutchcroft, J.E., Slavik, J.M., Lin, H., Watanabe, T., and Bierer, B.E. 1998. Uncoupling activation-dependent HS1 phosphorylation from nuclear factor of activated T cells transcriptional activation in Jurkat T cells: differential signaling through CD3 and the costimulatory receptors CD2 and CD28. J Immunol 161:4506-4512.
33. Tobin, G., Thunberg, U., Johnson, A., Thorn, I., Soderberg, O., Hultdin, M., Botling, J., Enblad, G., Sallstrom, J., Sundstrom, C., et al. 2002. Somatically mutated Ig V(H)3-21 genes characterize a new subset of chronic lymphocytic leukemia. Blood 99:2262-2264.
34. Tobin, G., Thunberg, U., Johnson, A., Eriksson, I., Soderberg, O., Karlsson, K., Merup, M., Juliusson, G., Vilpo, J., Enblad, G., et al. 2003. Chronic lymphocytic leukemias utilizing the VH3-21 gene display highly restricted Vlambda2-14 gene use and homologous CDR3s: implicating recognition of a common antigen epitope. Blood 101:4952-4957.
35. Goodnow, C.C. 1992. B-cell tolerance. Curr Opin Immunol 4:703-710.
36. Ghia, P., and Caligaris-Cappio, F. 2000. The indispensable role of microenvironment in the natural history of low-grade B-cell neoplasms. Adv Can Res 79:157-173.
37. Caligaris-Cappio, F. 1996. B-chronic lymphocytic leukemia: a malignancy of anti-self B cells. Blood 87:2615-2620.
38. Cheson, B.D., Bennett, J.M., Grever, M., Kay, N., Keating, M.J., O'Brien, S., and Rai, K.R. 1996. National Cancer Institute-sponsored Working Group guidelines for chronic lymphocytic leukemia: revised guidelines for diagnosis and treatment. In Blood. 4990-4997.
39. Ghia, P., Guida, G., Stella, S., Gottardi, D., Geuna, M., Strola, G., Scielzo, C., and Caligaris-Cappio, F. 2003. The pattern of CD38 expression defines a distinct subset of chronic lymphocytic leukemia (CLL) patients at risk of disease progression. Blood 101:1262-1269.
40. Binet, J.L., Lepoprier, M., Dighiero, G., Charron, D., D'Athis, P., Vaugier, G., Beral, H.M., Natali, J.C., Raphael, M., Nizet, B., et al. 1977. A clinical staging system for chronic lymphocytic leukemia: prognostic significance. Cancer 40:855-864.
41. Rai, K.R., and Han, T. 1990. Prognostic factors and clinical staging in chronic lymphocytic leukemia. Hematol Oncol Clin North Am 4:447-456.
42. Cheson, B.D., Bennett, J.M., Grever, M., Kay, N., Keating, M.J., O'Brien, S., and Rai, K.R. 1996. National Cancer Institute-sponsored Working Group guidelines for chronic lymphocytic leukemia: revised guidelines for diagnosis and treatment. Blood 87:4990-4997.
43. Ghia, P., Boussiotis, V.A., Schultze, J.L., Cardoso, A.A., Dorfman, D.M., Gribben, J.G., Freedman, A.S., and Nadler, L.M. 1998. Unbalanced expression of bcl-2 family proteins in follicular lymphoma: contribution of CD40 signaling in promoting survival. Blood 91:244-251.
44. Conti, A., Sanchez-Ruiz, Y., Bachi, A., Beretta, L., Grandi, E., Beltramo, M., and Alessio, M. 2004. Proteome study of human cerebrospinal fluid following traumatic brain injury indicates fibrin(ogen) degradation products as trauma-associated markers. J Neurotrauma 21:854-863.
45. Mortz, E., Krogh, T.N., Vorum, H., and Gorg, A. 2001. Improved silver staining protocols for high sensitivity protein identification using matrix-assisted laser desorption/ionization-time of flight analysis. Proteomics 1:1359-1363.
46. Shevchenko, A., Wilm, M., Vorm, O., and Mann, M. 1996. Mass spectrometric sequencing of proteins silver-stained polyacrylamide gels. Anal Chem 68:850-858.
47. Zhang, W., and Chait, B.T. 2000. ProFound: an expert system for protein identification using mass spectrometric peptide mapping information. Anal Chem 72:2482-2489

## Claims

1. *In vitro* use of a phosphorylated form of hematopoietic-lineage-cell-specific-protein-1 (HS1) as a prognostic or diagnostic marker for Chronic Lymphocytic Leukemia (CLL).

2. An *in vitro* method of determining the prognosis of an individual suffering from Chronic Lymphocytic Leukemia (CLL) comprising determining whether the individual's HS1 is in a phosphorylated form, and wherein an increased amount of the phosphorylated form relative to the unphosphorylated form indicates a poorer prognosis for the individual relative to a individual which has a decreased amount or same amount of the phosphorylated form relative to the unphosphorylated form.

3. An *in vitro* method of diagnosing Chronic Lymphocytic Leukemia (CLL) in an individual comprising determining whether the individual's HS1 is in a phosphorylated form, and wherein an increased amount of the phosphorylated form relative to a control indicates the presence of Chronic Lymphocytic Leukemia (CLL).

4. A method according to claim 2 or 3 wherein phosphorylated HS1 is detected using an antibody specific for the phosphorylated HS1.

5. A method according to claim 4 wherein the antibody is generatable against an HS1 sequence which comprises phosphorylated tyrosine 222, and/or 378, and/or 397.

6. A method according to any one of claims 2 to 5 wherein the method further comprises detecting the level of a further marker which correlates with the existence or prognosis of the disease.

7. Use of an antibody which specifically recognises phosphorylated HS1 for the preparation of a medicament for the treatment of Chronic Lymphocytic Leukemia (CLL).

8. The use according to claim 7 wherein the antibody is generatable against an HS1 sequence which comprises phosphorylated tyrosine 222, and/or 378, and/or 397.

9. A method of identifying an antagonist of HS1 phosphorylation for the prognosis or treatment of Chronic Lymphocytic Leukemia (CLL), the method comprising: (a) providing a candidate molecule, (b) incubating the candidate molecule with HS1 in a phosphorylating environment, and (c) detecting whether phosphorylation of HS1 occurs.

10. A method of identifying an agonist of HS1 dephosphorylation for the prognosis or treatment of Chronic Lymphocytic Leukemia (CLL), the method comprising: (a) providing a candidate molecule, (b) incubating the candidate molecule with phosphorylated HS1, and (c) detecting whether dephosphorylation of HS1 occurs.

11. An antibody which specifically recognises phosphorylated HS1 and which is generatable against an HS1 sequence which comprises phosphorylated tyrosine 222, and/or 378, and/or 397.

12. An antibody which specifically recognises phosphorylated HS1 for use in the treatment of Chronic Lymphocytic Leukemia (CLL).

13. An antibody according to claim 12 wherein the antibody is generatable against an HS1 sequence which comprises phosphorylated tyrosine 222, and/or 378, and/or 397.

## Patentansprüche

1. *In vitro*-Verwendung einer phosphorylierten Form eines für eine hämatopoetische Linie zellspezifischen Proteins 1 (HS1) als prognostizierenden oder diagnostischen Marker für die chronische lymphozytische Leukämie (CLL).

2. *In vitro*-Verfahren zur Bestimmung der Prognose eines Individuums, das an chronisch lymphozytischer Leukämie (CLL) leidet, bei dem man bestimmt, ob das HS1 des Individuums in einer phosphorylierten Form vorliegt, und wobei eine erhöhte Menge der phosphorylierten Form relativ zu der unphosphorylierten Form eine schlechtere Prognose für das Individuum relativ zu einem Individuum, das eine verringerte Menge oder die gleiche Menge der phosphorylierten Form relativ zu der unphosphorylierten Form aufweist, anzeigt.

3. *In vitro*-Verfahren zum Diagnostizieren von chronischer lymphozytischer Leukämie (CLL) in einem Individuum, bei dem man bestimmt, ob das HS1 des Individuums in einer phosphorylierten Form vorliegt, und wobei eine erhöhte Menge der phosphorylierten Form relativ zu einer Kontrolle das Vorliegen von chronisch lymphozytischer Leukämie (CLL) anzeigt.

4. Verfahren nach einem der Ansprüche 2 oder 3, wobei das phosphorylierte HS1 detektiert wird unter Verwendung eines Antikörpers, der für das phosphorylierte HS1 spezifisch ist.

5. Verfahren nach Anspruch 4, wobei der Antikörper gegen eine HS1-Sequenz generierbar ist, welche phosphoryliertes Tyrosin 222 und/oder 378 und/oder 397 umfasst.

6. Verfahren nach einem der Ansprüche 2 bis 5, wobei das Verfahren weiterhin umfasst, dass man den Gehalt an einem weiteren Marker, der mit dem Vorliegen oder der Prognose der Erkrankung korreliert, erfasst.

7. Verwendung eines Antikörpers, welcher phosphoryliertes HS1 spezifisch erkennt, für die Herstellung eines Medikaments für die Behandlung von chronisch lymphozytischer Leukämie (CLL).

8. Verwendung nach Anspruch 7, wobei der Antikörper gegen eine HS1-Sequenz generierbar ist, welche phosphoryliertes Tyrosin 222 und/oder 378 und/oder 397 umfasst.

9. Verfahren zum Identifizieren eines Antagonisten der HS1-Phosphorylierung für die Prognose oder Behandlung von chronisch lymphozytischer Leukämie (CLL), wobei das Verfahren umfasst: (a) Bereitstellen eines Kanditatenmoleküls, (b) Inkubieren des Kandidatenmoleküls mit HS1 in einer phosphorylierenden Umgebung und (c) Detektieren, ob Phosphorylierung von HS1 auftritt.

10. Verfahren zum Identifizieren eines Agonisten der HS1-Dephosphorylierung für die Prognose oder Behandlung von chronisch lymphozytischer Leukämie (CLL), wobei das Verfahren umfasst: (a) Bereitstellen eines Kandidatenmoleküls, (b) Inkubieren des Kandidatenmoleküls mit phosphoryliertem HS1 und (c) Detektieren, ob Dephosphorylierung von HS1 auftritt.

11. Antikörper, welcher phosphoryliertes HS1 spezifisch erkennt und welcher gegen eine HS1-Sequenz generierbar ist, welche phosphoryliertes Tyrosin 222 und/oder 378 und/oder 397 umfasst.

12. Antikörper, welcher phosphoryliertes HS1 spezifisch erkennt für die Verwendung bei der Behandlung von chronisch lymphozytischer Leukämie (CLL).

13. Antikörper nach Anspruch 12, wobei der Antikörper gegen eine HS1-Sequenz generierbar ist, welche phosphoryliertes Tyrosin 222 und/oder 378 und/oder 397 umfasst.

## Revendications

1. Utilisation *in vitro* d'une forme phosphorylée de la protéine 1 spécifique des cellules de la lignée hématopoïétique (HS1) en tant que marqueur pronostique ou diagnostique pour la leucémie lymphocytaire chronique (LLC).

2. Procédé *in vitro* de détermination du pronostic d'un individu souffrant d'une leucémie lymphocytaire chronique (LLC) comprenant la détermination si la HS1 de l'individu est sous une forme phosphorylée, et dans lequel une augmentation de la quantité de la forme phosphorylée par rapport à la forme non phosphorylée indique un pronostic plus médiocre pour l'individu par rapport à un individu qui présente une diminution de la quantité ou une même quantité de la forme phosphorylée par rapport à la forme non phosphorylée.

3. Procédé *in vitro* de diagnostic d'une leucémie lymphocytaire chronique (LLC) chez un individu comprenant la détermination si la HS1 de l'individu est sous une forme phosphorylée, et dans lequel une augmentation de la quantité de la forme phosphorylée par rapport à un contrôle indique la présence d'une leucémie lymphocytaire chronique (LLC).

4. Procédé selon la revendication 2 ou 3, dans lequel la HS1 phosphorylée est détectée en utilisant un anticorps spécifique de la HS1 phosphorylée.

5. Procédé selon la revendication 4, dans lequel l'anticorps peut être généré contre une séquence de la HS1 qui comprend la tyrosine phosphorylée 222 et/ou 378 et/ou 397.

6. Procédé selon l'une quelconque des revendications 2 à 5, où le procédé comprend en outre la détection d'un autre marqueur qui corrèle avec l'existence ou le pronostic de la maladie.

7. Utilisation d'un anticorps qui reconnaît spécifiquement la HS1 phosphorylée pour la préparation d'un médicament destiné au traitement d'une leucémie lymphocytaire chronique (LLC).

8. Utilisation selon la revendication 7, où l'anticorps peut être généré contre une séquence de la HS1 qui comprend la tyrosine phosphorylée 222 et/ou 378 et/ou 397.

9. Procédé d'identification d'un antagoniste de la phosphorylation de la HS1 pour le pronostic ou le traitement d'une leucémie lymphocytaire chronique (LLC), le procédé comprenant : (a) la fourniture d'une molécule candidate, (b) l'incubation de la molécule candidate avec la HS1 dans un environnement phosphorylant et (c) la détection si la phosphorylation de la SH1 se produit.

10. Procédé d'identification d'un agoniste de la déphosphorylation de la HS1 pour le pronostic ou le traitement d'une leucémie lymphocytaire chronique (LLC), le procédé comprenant : (a) la fourniture d'une molécule candidate, (b) l'incubation de la molécule candidate avec la HS1 phosphorylée et (c) la détection si la déphosphorylation de la HS1 se produit.

11. Anticorps qui reconnaît spécifiquement la HS 1 phosphorylée et qui peut être généré contre une séquence de la HS 1 qui comprend la tyrosine phosphorylée 222 et/ou 378 et/ou 397.

12. Anticorps qui reconnaît spécifiquement la HS1 phosphorylée à utiliser dans le traitement d'une leucémie lymphocytaire chronique (LLC).

13. Anticorps selon la revendication 12, où l'anticorps peut être généré contre une séquence de la HS1 qui comprend la tyrosine phosphorylée 222 et/ou 378 et/ou 397.
